(19) Europäisches Patentamt
European Patent Office
Office européen des brevets

(11) Veröffentlichungsnummer : **0 285 564 B1**

# (12) EUROPÄISCHE PATENTSCHRIFT

(45) Veröffentlichungstag der Patentschrift : **15.04.92 Patentblatt 92/16**

(21) Anmeldenummer : **88810192.0**

(22) Anmeldetag : **24.03.88**

(51) Int. Cl.$^5$ : **C07D 517/22,** C07D 517/06, C07D 495/22, C07D 495/06, C08K 5/48, C08K 5/45, H01B 1/20

## (54) Antistatische und elektrisch leitende Polymere und Formmassen.

(30) Priorität : **03.04.87 CH 1284/87**

(43) Veröffentlichungstag der Anmeldung : **05.10.88 Patentblatt 88/40**

(45) Bekanntmachung des Hinweises auf die Patenterteilung : **15.04.92 Patentblatt 92/16**

(84) Benannte Vertragsstaaten : **AT BE CH DE ES FR GB GR IT LI NL SE**

(56) Entgegenhaltungen :
**DE-A- 3 510 092**
**DE-A- 3 635 124**
**US-A- 3 403 165**
**US-A- 3 636 048**
**US-A- 4 384 025**

(73) Patentinhaber : **CIBA-GEIGY AG**
**Klybeckstrasse 141**
**CH-4002 Basel (CH)**

(72) Erfinder : **Finter, Jürgen, Dr.**
**Zasiusstrasse 100**
**W-7800 Freiburg/Br. (DE)**
Erfinder : **Hilti, Bruno, Dr.**
**Marschalkenstrasse 27**
**CH-4054 Basel (CH)**
Erfinder : **Mayer, Carl W., Dr.**
**Steingrubenweg 224**
**CH-4125 Riehen (CH)**
Erfinder : **Minder, Ernst**
**Oetlingerstrasse 44**
**CH-4057 Basel (CH)**
Erfinder : **Pfeiffer, Josef, Dr.**
**Brunnmattstrasse 32**
**CH-4106 Therwil (CH)**

EP 0 285 564 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine Kunststoffzusammensetzung , die ein gegebenenfalls substituiertes Tetrathio-, Tetraseleno- oder Tetratelluronaphthalin oder -tetracen enthält, eine Kunststoffzusammensetzung, die einen Charge-Transfer-Komplex (CT-Komplex) aus diesen Naphthalinen oder Tetracenen und einem Elektronenacceptor enthält, ein Verfahren zur Herstellung dieser Kunststoffzusammensetzung und deren Verwendung zur Herstellung antistatisch ausgerüsteter bzw. elektrisch leitender Formteile, Fäden, Fasern, Folien, Beschichtungen und Verbundstoffen.

In der DE-A-3005849 sind elektrisch leitende Formmassen aus einem thermoplastischen Kunststoff und einem CT-Komplex beschrieben, wobei diese CT-Komplexe faser- oder nadelförmig sind. Als Elektronendonator werden N, O und/oder S enthaltende Verbindungen und als Elektronenacceptor Polycyanverbindungen verwendet. Die Formmassen können durch Zugabe des Akzeptors zu einer Polymerlösung, in der der Donator gelöst ist, und anschliessendes Verdampfen des Lösungsmittels hergestellt werden. M. Kryszewski et al. beschreiben in Pure and Applied Chemistry, Vol. 56, No. 3, Seiten 355-368 (1984) elektrisch leitende Polymerzusammensetzungen, die als CT-Komplexe solche aus Tetrathiotetracen als Elektronendonator und Tetracyanochinodimethan, Tetracyanoethylen oder Chloranil als Elektronenacceptor enthalten. Die elektrische Leitfähigkeit dieser Systeme ist auf Grund der relativ niedrigen Leitfähigkeit der reinen CT-Komplexe ebenfalls gering.

Die Stabilität der CT-Komplexe mit Tetracyanochinodimethan ist gering. Es ist bekannt, dass diese CT-Komplexe gegen eine HCN-Abspaltung stabilisiert werden müssen, vgl. DE-A-3335513.

J.C. Stark et al. beschreiben in Organometallics, 3, S. 732-735 (1984), peri-dichalkogenierte Polyacene, von denen bestimmte Salze eine hohe elektrische Leitfähigkeit besitzen. In US-PS 4 384 025, US-PS 4 522 754, DE-OS 3 510 072, DE-OS 3 635 124 und EP-A-0 153 905 sind solche Halogenide beschrieben. Diese Halogenide weisen im allgemeinen einen Schmelzpunkt über 300°C auf. Ferner sind sie in organischen Lösungsmitteln praktisch unlöslich. Wegen dieser Eigenschaften können die Halogenide nur in Form von Pulvern in Polymere eingearbeitet werden. Solche Polymerzusammensetzungen weisen nur eine sehr geringe elektrische Leitfähigkeit auf, da die leitenden Teilchen in der Polymermatrix isoliert sind.

Ein Gegenstand vorliegender Erfindung ist eine Zusammensetzung, enthaltend

a) ein lineares, verzweigtes oder strukturell vernetztes Polymer, das gegenüber Komponente b) inert ist, und

b) eine Verbindung der Formel I oder Ia oder Mischungen davon,

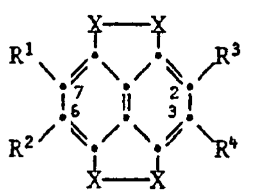 (I), 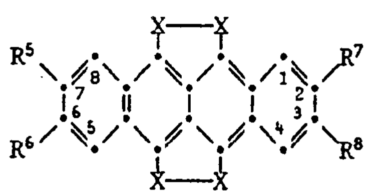 (Ia),

worin X für S, Se oder Te steht, $R^1$, $R^2$, $R^3$, und $R^4$ unabhängig voneinander ein Wasserstoffatom oder Cl bedeuten oder $R^1$ und $R^2$ sowie $R^3$ und $R^4$ zusammen je

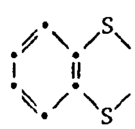 oder 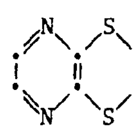 oder

$R^1$, $R^2$, $R^3$ und $R^4$ je Phenylthio, 4-Methyl- oder 4-Methoxyphenylthio oder 4-Pyridylthio darstellen, $R^5$, $R^6$, $R^7$ und $R^8$ unabhängig voneinander H oder F bedeuten, $R^5$ für $CH_3$ und $R^6$, $R^7$ und $R^8$ für H oder $R^5$, $R^6$, $R^7$ und $R^8$ für $CH_3$ stehen, $R^5$ und $R^6$ für $CH_3$ oder Cl und $R^7$ und $R^8$ für H stehen oder $R^5$ und $R^6$ für H, $R^7$ für $-COR^9$ und $R^8$ für H oder $-COR^9$, oder $R^5$ und $R^6$ für H und $R^7$ und $R^8$ zusammen für -CO-O-CO oder $-CO-NR^{10}-CO-$ stehen, worin $R^9$ Halogenid, -OH, $-NH_2$, $C_1$-$C_6$-Alkoxy, $C_2$-$C_6$-Hydroxyalkoxy, Benzyloxy, Phenoxy, Cyclopentyloxy, Cyclohexyloxy, $C_1$-$C_6$-Alkylamino oder Di($C_1$-$C_6$-Alkyl)amino darstellt, oder -OM ist, wobei M ein Metall- oder Ammoniumkation bedeutet, und $R^{10}$ H, $C_1$-$C_{18}$-Alkyl, Phenyl oder Benzyl ist.

Die Komponente b) ist bevorzugt in einer Menge von 0,01 bis 20 Gew.-%, besonders 0,05 bis 10 Gew.-% und insbesondere 0,1 bis 5 Gew.-% enthalten, bezogen auf das Polymer.

Einige Verbindungen der Komponente b) und ihre Herstellung sind in den zuvor erwähnten Publikationen beschrieben. Bevorzugte Verbindungen der Komponente b) sind Tetrathiotetracen, Tetraselenotetracen, 2-Fluor- oder 2,3-Difluortetraselenotetracen. Bevorzugte Mischungen sind solche aus Verbindungen der Formeln I und Ia, wobei die Verbindung der Formel Ia besonders 2,3,6,7-Tetrathiophenyl-tetrathionaphthalin ist. Mischungen dieser Tetracene mit einer Verbindung der Formel Ia enthalten bevorzugt 2,3,6,7-Tetrathiophenyl-1,4,5,8-tetrathionaphthalin.

Die neuen Verbindungen der Formeln II oder IIa

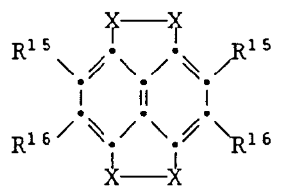 (II), 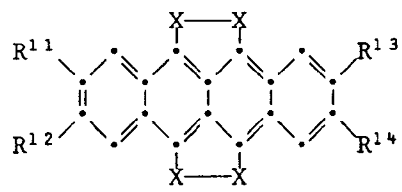 (IIa),

worin $R^{15}$ und $R^{16}$ je Phenylthio, 4-Methyl- oder 4-Methoxyphenylthio oder 4-Pyridylthio oder worin $R^{15}$ und $R^{16}$ zusammen

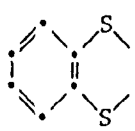 oder 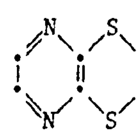

bedeuten, $R^{11}$ für $-CH_3$ und $R^{12}$, $R^{13}$ und $R^{14}$ für H, $R^{11}$ und $R^{12}$ für Cl oder $CH_3$ und $R^{13}$ und $R^{14}$ für H stehen oder $R^{11}$, $R^{12}$, $R^{13}$ und $R^{14}$ für $-CH_3$ oder F stehen, und X S, Se oder Te darstellt, sind ein weiterer Gegenstand der Erfindung. Ihre Herstellung kann wie nachfolgend beschrieben erfolgen:

a) Tetramethylierte Tetracene

Man setzt die bekannten Ausgangsverbindungen 4,5-Dimethylphthalsäureanhydrid und 2,3-Dimethyl-6,7-dihydroxynaphthalin in Gegenwart von $B_2O_3$ zu 2,3,8,9-Tetramethyl-5,12-dihydroxy-6,12-dioxo-tetracen (A) um. Diese Reaktion und die weitere Chlorierung und Reduktion zum in 5,6,11,12-Stellung tetrachlorierten Produkt sind in der DE-OS 3635124 beschrieben. Die Umsetzung mit $Na_2X_2$ führt zum entsprechenden tetrachalkogenierten Tetracen. In einer Variante kann das 2,3,8,9-Tetramethyl-5,5,6,11,12,12-hexachlordihydrotetracen (das bei der Chlorierung mit $PCl_5/POCl_3$ erhalten wird) mit 1 Val $Na_2Se_2$ und 2 Val $Na_2Se$ direkt zum entsprechenden Tetraselenotetracen umgesetzt werden. Die Verbindung A kann auch mit Dimethylsulfat zum 5,12-Dimethoxyderivat alkyliert werden [vgl. Chem. Pharm. Bull. 20(4), 827 (1972)]. Die Umsetzung dieses Derivates mit $P_4S_{10}$ in Tetrahydrofuran, die nachfolgene Oxidation mit $Br_2$ und anschliessende Reduktion mit $TiCl_3$ führt zum 2,3,8,9-Tetramethyl-5,6,11,12-tetrathiotetracen.

b) 2-Methyltetracene

Entsprechend der Vorschrift in Chem. Ber. 64, 1713 (1931) wird 2-Methyl-5,12,dioxo-dihydrotetracen erhalten. Die Reduktion mit Zn in alkalischer Lösung führt zum 2-Methyl-5,12-tetrahydrotetracen, das mit Chloranil zum 2-Methyltetracen dehydriert werden kann. Die Umsetzung mit S (siehe US-PS 3 723 417) ergibt das 2-Methyl5,6,11,12-tetrathiotetracen. Man kann auch wie in a) beschrieben das 2-Methyl-5,6,11,12-tetrachlortetracen herstellen und mit $Na_2X_2$ umsetzen.

c) Tetrafluortetracene

Gemäss der Vorschrift in Chem. Ber 31, 1159 und 1272 (1898) wird durch Kondensation von 2,3-Difluorphthalsäureanhydrid mit Bernsteinsäure und anschliessende Behandlung des Kondensationsproduktes mit Natriumethylat in Ethanol 2,3,8,9-Tetrafluor-5,12-dihydroxy-6,12-dioxo-tetracen (B) erhalten. Die weitere Umsetzung mit $PCl_5$ und anschliessend mit $SnCl_2/CH_3COOH$ zum 2,3,8,9-Tetrafluor-5,6,11,12-tetrachlortetracen erfolgt analog der Vorschrift in Zhuv. Org. Kim. 15(2), 391 (1979). Die Umsetzung mit $Na_2X_2$ ergibt die entsprechenden 2,3,8,9-Tetrafluortetrachalkogentetracene. Die Reduktion von Verbindung B mit Al in Cyclohexanol führt zum 2,3,8,9-Tetrafluortetracen, das mit Schwefel [siehe Bull. Soc. Chim. 15, 27 (1948)] zum 2,3,8,9-Tetrafluor-5,6,11,12-tetrathiotetracen reagiert.

d) Naphthaline

Ausgehend von bekannten (siehe US-PS 3 769 276) 2,3,6,7-Tetrachlortetrachalkogennaphthalinen können durch die Umsetzung mit den Kaliumsalzen von Thiophenol, 4-Methylthiophenol, 4-Methoxythiophenol, 4-Mercaptopyridin, 1,2-Benzodithiol bzw. Pyrazin-2,3-dithiol die entsprechenden

2,3,6,7-substituierten Tetrachalkogennaphthaline erhalten werden.

e) Dimethyl- und Dichlortetracene

Man verfährt analog wie unter a) beschrieben, setzt aber als Ausgangsverbindungen 4,5-Dimethyl- bzw. 4,5-Dichlorphthalsäureanhydrid mit 6,7-Dihydroxynaphthalin um und chloriert mit $PCl_5/POCl_3$.

In den Formeln I, Ia, II und IIa stellt X bevorzugt S oder Se dar. $R^9$ ist als Halogenid besonders Chlorid.

In dem Rest -OM kann M ein Metall- oder Ammoniumkation sein. Als Metallkation kommen insbesondere solche der Alkali- und Erdalkalimetalle in Frage, z.B. $Li^{\oplus}$, $Na^{\oplus}$, $K^{\oplus}$, $Mg^{2\oplus}$, $Ca^{2\oplus}$, $Sr^{2\oplus}$ und $Ba^{2\oplus}$. Ferner sind $Zn^{2\oplus}$ und $Cd^{2\oplus}$ geeignet. Als Ammoniumkationen kommen z.B. $NH^{4\oplus}$ und primäres, sekundäres, tertiäres oder quaternäres Ammonium in Frage, die vorzugsweise $C_1$-$C_{12}$-Alkyl-, Cyclohexyl-, Cyclopentyl-, Phenyl- oder Benzylgruppen enthalten kann. Die Ammoniumkationen können sich auch von 5- oder 6-gliedrigen heterocyclischen Aminen ableiten, z.B. Piperidin, Pyrrol und Morpholin.

$R^{10}$ enthält als Alkyl bevorzugt 1 bis 12 und besonders 1 bis 6 C-Atome. Beispiele für Alkyl, das linear oder verzweigt sein kann, sind: Methyl, Ethyl, n- oder i-Propyl, n-, i- oder t-Butyl, Pentyl, Hexyl, 2-Ethylhexyl, Heptyl, Octyl, Nonyl, Decyl, Undecyl und Dodecyl. Beispiele für Alkoxy und Hydroxyalkoxy sind: Methoxy, Ethoxy, Propoxy, Butoxy, Pentoxy, Hexoxy, β-Hydroxyethoxy, γ-Hydroxypropoxy, δ-Hydroxybutoxy und ω-Hydroxyhexoxy.

Die Polymeren der Komponente a) sind gegenüber den Verbindungen der Komponente b) inert. Die Polymeren enthalten daher bevorzugt im wesentlichen keine stark sauren Gruppen, z.B. Carboxygruppen, bzw. stark basische Gruppen, z.B. primäre oder sekundäre Amin- oder Hydroxylgruppen. Bei den Polymeren kann es sich z.B. um Duroplaste, Thermoplaste oder Elastomere handeln.

Beispiele für Polymere sind:

1. Polymere von Mono- und Diolefinen, beispielsweise Polypropylen, Polyisobutylen, Polybuten-1, Polymethylpenten-1, Polyisopren oder Polybutadien sowie Polymerisate von Cycloolefinen wie z.B. von Cyclopenten oder Norbornen; ferner Polyethylen, z.B. Polyethylen hoher Dichte (HDPE), Polyethylen niederer Dichte (LDPE), lineares Polyethylen niederer Dichte (LLDPE).
2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).
3. Copolymere von Mono- und Diolefinen untereinander oder mit anderen Vinylmonomeren, wie z.B. Ethylen-Propylen-Copolymere, Propylen-Buten-1-Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere, sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäureester-Copolymere, LLDPE/Ethylen-Vinylacetat-Copolymere und LLDPE/Ethylen-Acrylsäureester-Copolymere.
4. Polystyrol, Poly-(p-methylstyrol), Poly-(α-methylstyrol).
5. Copolymere von Styrol oder α-Methylstyrol mit Dienen oder Acrylderivaten, wie z.B. Styrol-Butadien, Styrol-Acrylnitril, Styrol-Alkylmethacrylat, Styrol-Butadien-Alkylacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol, Styrol-Ethylen/Propylen-Styrol oder Styrol-4-Vinylpyridin-Styrol.
6. Pfropfcopolymere von Styrol oder α-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z.B. als sogenannte ABS-, MBS-, ASA- oder AES-Polymere bekannt sind.
7. Halogenhaltige Polymere, wie z.B. Polychloropren, Chlorkautschuk, chloriertes oder chlorsulfoniertes Polyethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie

deren Copolymere, wie Vinylchlorid-Vinylidenchlorid, Vinylchlorid-Vinylacetat oder Vinylidenchlorid-Vinylacetat.

8. Polymere, die sich von Derivaten α,β-ungesättigter Säuren ableiten, wie Polyacrylate, Polymethacrylate und Polyacrylnitrile.

9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere, Acrylnitril-Alkylmethacrylat-Butadien-Terpolymere oder Alkylmethacrylat-4-Vinylpyridin-Copolymere.

10. Polymere, die sich von Acylderivaten oder Acetalen ungesättigter Alkohole ableiten, wie Polyvinylacetat, -stearat, -benzoat, -maleat, Polyvinylbutyral, Polyallylphthalat; sowie deren Copolymere mit in Punkt 1 genannten Olefinen.

11. Homo- und Copolymere von cyclischen Ethern, wie Polyalkylenglykole, Polyethylenoxyd, Polypropylenoxyd oder Polybutylenglykol.

12. Polyacetale, wie Polyoxymethylen, sowie solche Polyoxymethylene, die Comonomere, wie z.B. Ethylenoxid, enthalten; Polyacetale, die mit thermoplastischen Polyurethanen, Acrylaten oder MBS modifiziert sind.

13. Polyphenylenoxide und -sulfide und deren Mischungen mit Styrolpolymeren.

14. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.

15. Polyharnstoffe, Polyimide und Polybenzimidazole. Unter den Polyimiden sind besonders lösliche Polyimide bevorzugt wie sie z.B. in DE-AS 1962588, EP-A-132221, EP-A-134752, EP-A-162017, EP-A-181837 und EP-A-182745 beschrieben sind.

16. Polycarbonate, Polyester, z.B. Polyalkylenterephthalate, und Polyestercarbonate.

17. Polysulfone, Polyethersulfone und Polyetherketone.

18. Polyvinylcarbazol.

19. Vernetzbare Acrylharze, die sich von substituierten Acrylsäureestern ableiten, wie z.B. von Epoxyacrylaten, Urethan-acrylaten oder Polyester-acrylaten, z.B. Ester von Polyolen wie Glykolen, Trimethylolpropan, Pentaerythrit oder Polyepoxiden.

20. Vernetzte Epoxidharze, die sich von Polyepoxiden ableiten, z.B. von Bis-glycidylethern oder von cycloaliphatischen Diepoxiden. Sie können z.B. mit Anhydriden, thermisch unter Verwendung von Härtungsbeschleunigern oder durch Einwirkung von UV-Strahlung vernetzt sein.

21. Polymerhomolog chemisch abgewandelte Derivate von Cellulose, wie Celluloseacetate, -propionate und -butyrate, bzw. die Celluloseether, wie Methylcellulose.

22. Mischungen (Polyblends) der vorgenannten Polymeren, wie z.B. PP/EPDM, PVC/EVA, PVC/ABS, PVC/MBS, PC/ABS, PC/ASA, PC/PBT, PVC/CPE, PVC/Acrylate, POM/thermoplastisches PUR, PC/thermoplastisches PUR, POM/Acrylat, POM/MBS, PPO/HIPS.

23. Mit Schwefel vernetzte (vulkanisierte) Produkte aus Doppelbindungen enthaltenden Polymeren wie z.B. Naturkautschuk, Synthesekautschuk, Butadien- bzw. Isoprenpolymerisate oder -copolymerisate.

Eine bevorzugte Gruppe von thermoplastischen Polymeren sind Polyolefine, Polystyrol, Polyvinylchlorid, Polyvinylidenchlorid, Polyvinylidenfluorid, Polyacrylate, Polymethacrylate, Polycarbonate, aromatische Polysulfone, aromatische Polyether, aromatische Polyethersulfone, Polyimide und Polyvinylcarbazol.

Die erfindungsgemässe Zusammensetzung kann zusätzlich ein Lösungsmittel für ein lösliches Polymer und die Komponente b) enthalten. Geeignete Lösungsmittel sind z.B. polare, aprotische Lösungsmittel, die alleine oder in Mischungen aus mindestens zwei Lösungsmitteln verwendet werden können. Beispiele sind: Ether wie Dibutylether, Tetrahydrofuran, Dioxan, Methylenglykol, Dimethylethylenglykol, Dimethyldiethylenglykol, Diethyldiethylenglykol, Dimethyltriethylenglykol, halogenierte Kohlenwasserstoffe wie Methylenchlorid, Chloroform, 1,2-Dichlorethan, 1,1,1,-Trichlorethan, 1,1,2,2-Tetrachlorethan, Carbonsäureester und Lactone wie Essigsäureethylester, Propionsäuremethylester, Benzoesäureethylester, 2-Methoxyethylacetat, γ-Butyrolacton, δ-Valerolacton und Pivalolacton, Carbonsäureamide und Lactame wie N-Methylformamid, N,N-Dimethylformamid, N,N-Diethylformamid, N,N-Dimethylacetamid, N,N-Diethylacetamid, γ-Butyrolactam, ε-Caprolactam, N-Methylpyrrolidon, N-Acetylpyrrolidon, N-Methylcaprolactam, Tetramethylharnstoff, Hexamethylphosphorsäuretriamid, Sulfoxide wie Dimethylsulfoxid, Sulfone wie Dimethylsulfon, Diethylsulfon, Trimethylensulfon, Tetramethylensulfon, N-Methylpyrrolidin, N-Methylpiperidin, N-Methylmorpholin, substituierte Benzole wie Benzonitril, Chlorbenzol, o-Dichlorbenzol, 1,2,4-Trichlorbenzol, Nitrobenzol, Toluol und Xylol.

Die erfindungsgemässe Zusammensetzung kann zusätzlich für die Verarbeitung und Anwendung benötigte Hilfsmittel enthalten, z.B. Weichmacher, Verlaufmittel, Formtrennmittel, Füllstoffe, Flammschutzmittel, Antioxidantien und Lichtschutzmittel, Stabilisatoren, Farbstoffe und Pigmente.

Die erfindungsgemässe Zusammensetzung kann zusätzlich einen Elektronenacceptor, z.B. elementares Halogen ($Cl_2$, $Br_2$, $I_2$) enthalten, oder eine halogenhaltige organische Verbindung, welche gegebenenfalls unter Zufuhr von Energie Halogen abspaltet und mit einer Verbindung der Formel I oder Ia (Donor) einen Charge-Transferkomplex (Donor)(Halogen)$_x$ bildet, wobei 0,3 >x< 0,9 ist. Bevorzugt ist 0,3 >x< 0,8 und besonders x gleich 0,5 für Cl und Br und x = 0,76 für I. Bei der Energie kann es sich z.B. um thermische Energie oder Strahlungsenergie handeln. Im Fall von Strahlungsenergie kann z.B. bildmässig durch eine Maske oder mittels bildmässiger Steuerung eines Lichtstrahls oder flächenmässig bestrahlt werden. Thermische Energie bedeutet zum Beispiel eine Temperatur von Raumtemperatur bis 350°C, insbesondere 50 bis 200°C. Das Mengenverhältnis von Elektronenacceptor zu Komponente b) beträgt bevorzugt 10:1 bis 1:5, besonders 5:1 bis 1:3 und insbesondere 2:1 bis 1:2. Der Elektronenacceptor, besonders die halogenhaltige organische Verbindung kann aber auch in erheblich höheren Mengen enthalten sein und gleichzeitig als Lösungsmittel für das thermoplastische Polymer und die Komponente b) dienen, wenn es sich z.B. um eine flüssige halogenhaltige organische Verbindung handelt. Die organische Verbindung kann auch fest sein und sie soll mit dem Polymer mischbar und verträglich sein.

Bei der halogen-, besonders Cl-, Br- oder I-haltigen organischen Verbindung kann es sich um eine halogenierte, gesättigte oder ungesättigte, aliphatische, cycloaliphatische, aliphatisch-heterocyclische, aromatische oder heteroaromatische organische Verbindung handeln, die durch -CN, HO-, =O, $C_1$-$C_4$-Alkyl, $C_1$-$C_4$-Alkoxy, -CO-$C_1$-$C_4$-Alkyl, -COO$C_1$-$C_4$-Alkyl substituiert sein können. Die Halogenverbindungen können einzeln oder in Gemischen eingesetzt werden. Die organische Verbindung ist bevorzugt chloriert und/oder bromiert. Die Verbindungen können einfach halogeniert sein, wie z.B. N-bromierte oder N-chlorierte Dicarbonsäureimide. C-halogenierte Verbindungen weisen zweckmässig einen höheren Halogenierungsgrad auf; bevorzugt sind diese Verbindungen zu mindestens 80 % C-halogeniert, besonders C-bromiert und/oder C-chloriert. Verbindungen, deren Halogenatome durch elektronenziehende Gruppen aktiviert sind, sind besonders günstig.

Beispiele für halogenierte organische Verbindungen sind Tetrabrommethan, Bromoform, Trichlorbrommethan, Hexachlorpropen, Hexachlorcyclopropan, Hexachlorcyclopentadien, Hexachlorethan, N-Chlorsuccinimid, Octachlorpropan, n-Octachlorbutan, n-Decachlorbutan, Tetrabromethan, Hexabromethan, Tetrabrom-o-benzochinon, 2,4,4,6-Tetrabrom-2,5-cyclohexadienon, Hexabrombenzol, Chloranil, Hexachloraceton, 1,4,5,6,7,7-Hexachlor-5-norbornen-2,3-dicarbonsäure, 1,2,5,6,9,10-Hexabromcyclododecan, Tetrachlorethylen, Perchlorcyclopentadien, Perchlorbutadien, Dichloracetaldehyd-diethylacetal, 1,4-Dichlor-2-buten, 1,3-Dichlor-2-buten, 3,4-Dichlor-1-buten, Tetrachlorcyclopropen, 1,3-Dichloraceton, 2,3,5,6-Hexachlor-p-xylol, 1,4-Bis(trichlormethyl)-benzol, 1,3-Dibrompropan, 1,6-Dibromhexan, 3-Chlorpropionsäureethylester, 3-Chlortoluol, 2-Chlorpropionsäuremethylester, 2-Chloracrylnitril, Trichloressigsäureethylester, 1,2,3-Trichlorpropan, 1,1,2-Trichlorethan, Chlorameisensäurebutylester, Trichlorethylen, 2,3-Dichlormaleinsäureanhydrid, 1,12-Dibromdodecan, $\alpha,\alpha'$-Dibromp-xylol, $\alpha,\alpha'$-Dichlor-o-xylol, Phenacylchlorid oder -bromid, 1,10-Dibromdecan, $\alpha,\alpha'$-Dichlor-p-xylol, $\alpha,\alpha'$-Dibrom-m-xylol, Jodacetonitril, 2,3-Dichlor-5,6-dicyanobenzochinon, 2,3-Dichlorpropionsäuremethylester, 1-Brom-2-chlorethan, 1-Brom-2-chlorpropan, Chlorameisensäure-2-bromethylester, Jodessigsäureethylester, N-Chlor-, N-Brom- oder N-Jodsuccinimid oder -phthalsäureimid, oder Mischungen hiervon.

Weitere geeignete Elektronenacceptoren sind z.B. $O_2$ oder Salze von oxidativ wirkenden Kationen mit nicht nucleophilen Anionen, wie z.B. Halogen ($F^\ominus$, $Cl^\ominus$), $BF_4^\ominus$, $SbF_6^\ominus$, $AsF_6^\ominus$ und $PF_6^\ominus$. Beispiele für Kationen sind solche von Uebergangsmetallen oder seltenen Erdmetallen [Fe(III), Co(III), Ce(IV)] oder Nichtmetallkationen wie z.B. $NO^\oplus$. Beispiele sind $NOBF_4$, $FeCl_3$ oder $Co(PF_6)_3$.

Die Herstellung der erfindungsgemässen Zusammensetzung erfolgt durch einfaches Vermischen der Komponenten, gegebenenfalls unter Mitverwendung eines Lösungsmittels. Mit dem Vermischen kann auch eine Formgebung verbunden sein, unter Anwendung bekannter Methoden wie z.B. Giessen, Spritzgiessen, Kalandrieren oder Extrusion. Bei duroplastischen Harzen wird die Verbindung der Formel I oder Ia zweckmässig einer härtbaren Komponente (z.B. Harz oder Härter) zugegeben, und die Aushärtung zum duroplastischen Polymer nach Vermischen der Komponenten vorgenommen.

Aus den erfindungsgemässen Zusammensetzungen können auf einfache Weise einen Charge-Transfer-Komplex (CT-Komplex) enthaltende Zusammensetzungen hergestellt werden. Ein weiterer Gegenstand der Erfindung ist eine Zusammensetzung, enthaltend

a) ein lineares, verzweigtes oder strukturell vernetztes Polymer und

b) ein Netzwerk aus elektrisch leitenden Kristallnadeln in der Polymermatrix von mindestens einem CT-Komplex, der aus mindestens einer Verbindung der Formeln I oder Ia in Form von Chloriden, Bromiden oder Iodiden, oder mit $O_2$ oder einem Salz eines oxidativ wirkenden Kations mit nicht nukleophilen Anionen als Elektronenakzeptor.

Der CT-Komplex kann z.B. in einer Menge von 0,01 bis 20, bevorzugt 0,1 bis 10 und besonders 0,1 bis 5 Gew.-% enthalten sein, bezogen auf das Polymer. Bei dem CT-Komplex handelt es sich bevorzugt um Chloride, Bromide oder Iodide von Verbindungen der Formeln I oder Ia. Im übrigen gelten die Bevorzugungen für die zuvor beschriebenen Zusammensetzungen.

Die Herstellung der CT-Komplexe enthaltenden Zusammensetzung ist dadurch gekennzeichnet, dass man auf eine erfindungsgemässe Zusammensetzung, enthaltend

a) ein lineares, verzweigtes oder strukturell vernetztes Polymer, und

b) mindestens eine Verbindung der Formeln I oder Ia, $O_2$, ein gas- oder dampfförmiges Halogenierungsmittel, eine organische halogenhaltige Verbindung, welche bei Energiezufuhr Halogen bildet, oder ein Salz eines oxidativ wirkenden Kations mit nicht nukleophilen Anionen, als Elektronenacceptor einwirken lässt.

Bei dem Elektronenacceptor handelt es sich um $O_2$, ein gas- oder dampfförmiges Halogenierungsmittel, eine organische halogenhaltige Verbindung, welche bei Energiezufuhr Halogen bildet, besonders Cl, Br und/oder I, oder um ein Salz eines oxidativ wirkenden Kations mit nicht nukleophilen Anionen.

Das Einwirken des Elektronenacceptors erfolgt zweckmässig bei Temperaturen von z.B. Raumtemperatur bis 350°C, vorzugsweise 50 bis 200°C.

In einer erfindungsgemässen Ausführungsform lässt man einen gas-oder dampfförmigen Elektronenacceptor, z.B. $O_2$, oder ein Halogenierungsmittel wie z.B. $XeF_2$, $Cl_2$, $Br_2$ oder $I_2$ auf die Zusammensetzung einwirken.

Eine bevorzugte Ausführungsform des Verfahrens ist dadurch gekennzeichnet, dass der Elektronenacceptor eine der Zusammensetzung einverleibte halogenhaltige organische Verbindung ist, die durch Energiezufuhr, z.B. Erwärmen Halogenid, z.B. Cl, Br und/oder I bildet. Erwärmen kann eine Temperatur bis 350°C, vorzugsweise 50-200°C bedeuten.

In einer anderen Ausführungsform vermischt man die Zusammensetzung mit einem Salz eines oxidativ wirkenden Kations mit nicht nukleophilen Anionen und führt dieser Mischung Energie, z.B. Wärme zu.

Die organische halogenhaltige Verbindung oder ein Salz kann gleichzeitig oder nach dem Vermischen eines Polymers mit einer Verbindung der Formel I und/oder Ia zugegeben werden. Gleichzeitig mit dem Vermischen kann eine Formgebung verbunden sein, z.B. durch Giessen, Spritzgiessen, Extrudieren und Kalandrieren. Bei der Herstellung duroplastischer Polymerer wird die organische halogenhaltige Verbindung oder ein Salz zweckmässig vor der Härtung bzw. Polymerisation einer Komponente, bei Epoxidharzen z.B. dem Epoxidharz, einverleibt. Nach der gewünschten Formgebung kann dann die Härtung bzw. Polymerisation vorgenommen werden.

Die zur Freisetzung von Halogenid, z.B. Cl, Br und/oder I und zur Bildung von CT-Komplexen notwendige Temperatur kann bei der Formgebung und bei Duroplasten mit der Härtung bzw. Polymerisation erzielt werden. Die Erwärmung kann aber auch nach der Formgebung vorgenommen werden. Bei der Mitverwendung von Lösungsmitteln wird vorteilhaft mit dem Erwärmen das Lösungsmittel entfernt.

Die erfindungsgemässen, einen CT-Komplex enthaltenden Zusammensetzungen stellen wertvolle Formmassen dar, aus denen nach üblichen Verfahren Gebrauchsartikel wie z.B. Formteile, Folien, Filme, Fäden, Fasern oder Beschichtungen hergestellt werden können, die antistatisch ausgerüstet oder elektrisch leitend sind.

Ein weiterer Gegenstand der Erfindung ist die Verwendung der beschriebenen, einen CT-Komplex enthaltenden Zusammensetzung zur Herstellung von a) antistatisch ausgerüsteten und/oder b) elektrisch leitenden Formteilen, Folien, Fäden, Fasern, Beschichtungen oder Verbundstoffen.

Ein bevorzugter Anwendungsbereich ist die Herstellung von Beschichtungen oder Folien durch z.B. Extrusion oder nach Giess- bzw. Streichverfahren. Sie können für die elektrostatische Abschirmung von Bauteilen verwendet werden. Die Folien stellen flexible elektrische Leiter dar, aus denen Elektroden z.B. für Displayelemente hergestellt werden können. Je nach verwendetem Polymer sind auch transparente Ausführungsformen möglich.

Die erfindungsgemässen, einen CT-Komplex enthaltenden Zusammensetzungen zeichnen sich durch eine hohe chemische Stabilität und Temperaturbeständigkeit und eine geringe Migration der CT-Komplexe aus. Ferner werden überraschend hohe Leitfähigkeiten erzielt, die bis zu 25 % der Leitfähigkeit der reinen CT-Komplexe betragen können. Die CT-Komplexe bilden ein Netzwerk aus elektrisch leitenden Kristallnadeln in der Polymermatrix.

Die nachfolgenden Beispiele erläutern die Erfindungen näher.

A) Herstellung von Ausgangsprodukten.

a1) Herstellung von 2,3,8,9-Tetramethyl-5,6,11,12-tetrathiotetracen

Man gibt 0,6 g (1,6 mMol) 2,3,8,9-Tetramethyl-5,11-dimethoxytetracen-6,12-chinon, 0,75 g $P_4S_{10}$ (1,68 mMol), 0,1 g Schwefel und 50 ml Dioxan zusammen und erhitzt während 22 Stunden am Rückfluss. Der Niederschlag wird heiss abfiltriert, mit Dioxan und dann mit Chloroform gewaschen und im Hochvakuum getrocknet. Man erhält 0,6 g Produkt, das in 100 ml Ameisensäure 5 Minuten im Ultraschallbad verrührt wird. Es werden 10 ml einprozentige $Br_2$-Lösung zugetropft, am Rückfluss erhitzt und weitere 7 ml $Br_2$-Lösung zugetropft. Die unlöslichen Anteile werden abfiltriert. Die Lösung wird mit 500 ml Wasser verdünnt und mit saurer $TiCl_3$-Lösung reduziert (Merck, ca. 15 % $TiCl_3$ in 10 %-iger Salzsäure). Der gebildete Niederschlag wird unter Argonatmosphäre abgesaugt, mit Wasser gewaschen und am Hochvakuum getrocknet. Man erhält 0,4 g (61 % der Theorie) Rohprodukt, das bei 270°C/0,13 Pascal sublimiert wird. Man erhält 70 mg 2,3,8,9-Tetramethyl-5,6,11,12-tetrathiotetracen als schwarze Kristalle. Massenspektrum: $M^+$ = 408. $\lambda_{max}$ (1,2,4-Trichlorbenzol): 691 nm.

a2) Herstellung von 2,3,8,9-Tetramethyl-5,6,11,12-tetraselenotetracen

1) Chlorierung von 2,3,8,9-Tetramethyl-6,11-dihydroxi-tetracenchinon-5,12

In einem 350 ml Sulfierkolben mit Gaseinleitungsrohr, Thermometer und Rückflusskühler werden unter leichtem Argonfluss 40,99 g Phosphorpentachlorid in 102,5 ml Phosphorylchlorid bei 75°C gelöst. Zu dieser klaren gelblichen Lösung werden dann 12,2 g (0,035 Mol) 2,3,8,9-Tetramethyl-6,11-dihydroxi-tetracenchinon-5,12 zugegeben; die rote Suspension wird für 4 Stunden am Rückfluss erhitzt. Dabei entsteht allmählich eine Suspension von beiger Farbe. Das auf Raumtemperatur abgekühlte Reaktionsgemisch wird filtriert, der beige Festkörper mit Essigsäure gut gewaschen und bei 50°C ($1,3 \cdot 10^{-2}$ mbar) getrocknet. Man erhält 12,4 g 2,3,8,9-Tetramethyl-5,5,6,11,12,12-hexachlortetracen (71 % Ausbeute). Massenspektrum: $M^+$ = 490 (6Cl).

2) Herstellung des 2,3,8,9-Tetramethyl-5,6,11,12-tetraselenotetracens

In einem 250 ml Dreihalskolben mit Gaseinleitungsrohr und Rückflusskühler werden 1,58 g (20 mVal) Selen (Ventron, 99,9999 %) und 0,69 g Na (30 mVal) in 150 ml (über A4-Molekularsieb getrocknetem) Dimethylformamid (DMF) unter leichtem Argonfluss bei 130°C zu einem Gemisch von $Na_2Se$ (20 mMol) und $Na_2Se_2$ (10 mMol) umgesetzt. Bei der gleichen Temperatur werden dann 2,47 g (5 mMol) 2,3,8,9-Tetramethyl-5,5,6,11,12,12-hexachlortetracen zugegeben. Die Farbe des Reaktionsgemisches schlägt sofort nach tiefgrün um. Nach einer Reaktionszeit von 20 Stunden wird auf Raumtemperatur abgekühlt und der schwarze, blaugrün schillernde Festkörper abgenutscht, mit DMF, Wasser und Aceton gewaschen (je 3 Portionen à 50 ml) und bei 50°C ($1,3 \cdot 10^{-2}$ mbar) getrocknet. Rohausbeute 2,06 g (69 %). Dieses Rohprodukt wird in 200 ml wasserfreier Ameisensäure aufgeschlämmt und durch Einleiten von Sauerstoff oxidiert. Die blaue Lösung des Kationradikals wird filtriert und mit Ti-III-chlorid-Lösung (15 % $TiCl_3$ in 10 % Salzsäure) versetzt, bis die Lösung leicht rot-violett gefärbt bleibt. Dabei fällt das 2,3,8,9-Tetramethyl-5,6,11,12-tetraselenotetracen als flockiger giftgrüner Niederschlag aus. Es wird filtriert und der Festkörper mit soviel Wasser gewaschen bis das Eluat farblos und neutral ist. Das bei 50°C ($1,3 \cdot 10^{-2}$ mbar) getrocknete Produkt wird bei ca. 300°C und $1,3 \cdot 10^{-3}$ mbar sublimiert. Es entstehen schwarze Nädelchen. $\lambda_{max}$ (1,2,4-Trichlorbenzol): 693, 639, 462 nm.
Massenspektrum: $M^+$ = 596 (4Se). Die Fragmentierung steht mit der erwarteten Struktur im Einklang.

b) Herstellung von 2-Methyl-5,6,11,12-tetrathiotetracen

1,7 g (7 mMol) 2-Methyltetracen und 3,4 g (100 mMol) Schwefel werden in 40 ml frisch destilliertem und über einem Molekularsieb getrocknetem Dimethylformamid während 4 Stunden am Rückfluss gehalten. Nach dem Abkühlen wird der schwarz-grüne Niederschlag abfiltriert. Man erhält 2 g (78 % der Theorie) Rohpodukt, das aus 200 ml Chlorbenzol umkristallisiert wird.

Es ergeben sich 1,2 g 2-Methyl-5,6,11,12-tetrathiotetracen als schwarze Kristallnadeln.
Massenspektrum: $M^+$ = 366. $\lambda_{max}$ (1,2,4-Trichlorbenzol): 705 nm.

c) Herstellung von 2,3,8,9-Tetrafluor-5,6,11,12-tetrathiotetracen

Unter Argon werden 30 mg 2,3,8,9-Tetrafluortetracen (0,1 mMol), 22 mg Schwefel (0,68 mVal) in 5 ml 1,2,4-Trichlorbenzol während 72 Std. am Rückfluss erhitzt. Nach dem Abkühlen der Reaktionslösung wird das ausgefallene 2,3,8,9-Tetrafluor-5,6,11,12-tetrathiotetracen abfiltriert und mit Trichlorbenzol und Aceton gewaschen, bis das Filtrat farblos ist. Man erhält 25 mg schwarz-grünes Pulver (58,9 % der Theorie).
$\lambda_{max}$ (1,2,4-Trichlorbenzol): 702 nm. Massenspektrum: $M^+$ = 424.

d) Herstellung von 2,3,8,9-Tetrafluor-5,6,11,12-tetraselenotetracen

In einem 50 ml Kolben werden unter Argon und Rühren 37 mg Na (1,6 mMol) und 125 mg Se (1,55 mMol) in 10 ml Dimethylformamid (DMF, 2 x destilliert, über Molekularsieb getrocknet) bei 110°C Innentemperatur zu $Na_2Se_2$ umgesetzt. Die Reaktionszeit beträgt 1 Stunde. Es entsteht eine rote Lösung mit wenig rotbraunem Niederschlag.
Danach werden 162 mg 2,3,8,9-Tetrafluor-5,6,11,12-tetrachlortetracen (0,58 mMol) aufgeschlämmt in 10 ml DMF, in die auf 55°C abgekühlte $Na_2Se_2$-Lösung gegeben. Während 20 Stunden wird bei dieser Temperatur gehalten. Darauf wird der Niederschlag abgesaugt, mit DMF, $CHCl_3$, Benzol und Aceton (je 3 Portionen ca. ~10 ml) gewaschen und am Hochvakuum bei 50°C getrocknet. Man erhält 263 mg Rohprodukt (>100 % der Theorie; enthält noch NaCl).
Das Rohprodukt wird in 50 ml Ameisensäure aufgeschlämmt und mit Sauerstoff oxidiert. Es entsteht eine blaue Lösung, welche filtriert wird. Anschliessend wird die Lösung mit dem dreifachen Volumen Wasser verdünnt und mit $TiCl_3$-Lösung (Merck: ca. 15 % $TiCl_3$ in 10 %-igen Salzsäure) reduziert, bis die rotviolette Farbe des Reduktionsmittels erhalten bleibt. Das Produkt fällt als grüner flockiger Niederschlag aus. Es wird abgenutscht, mit Wasser gewaschen, am Hochvakuum bei 50°C getrocknet. Man erhält 160 mg vorgereinigtes Produkt (68 % d.Th.).

Das vorgereinigte Produkt wird bei 270°C/0,13 Pascal sublimiert. Es ergeben sich 51,5 mg schwarz-grüne Nadeln.
Massenspektrum: $M^+$ = 616 (Cluster mit 4 Se). $\lambda_{max}$ : 712 nm.

e) Herstellung von 2,3-Dichlor-5,6,11,12-tetraselenotetracen

In analoger Weise zu Beispiel d) werden in 40 ml DMF 408 mg $Na_2Se_2$ (2 mMol) hergestellt und 432 mg (1 mMol) 2,3,5,6,11,12-Hexachlortetracen in 30 ml DMF dazugegeben. Während 24 Stunden wird bei 80°C gehalten. Danach wird der schwarze Niederschlag abgesaugt, mit DMF, $CHCl_3$, Benzol, Aceton und Wasser (je 3 Portionen à 20 ml) gewaschen. Man erhält 600 mg Rohprodukt, das nach Trocknen bei 290°C ($1{,}3 \cdot 10^{-2}$ mbar) sublimiert wird; man erhält so 214 mg (35,2 %) schwarze Nädelchen von 2,3-Dichlor-5,6,11,12-tetraselenotetracen. $\lambda_{max}$ in Trichlorbenzol: 746, 690, 540 nm.
Massenspektrum: $M^+$ = 608 (4Se, 2Cl). Die Fragmentierung steht mit der Struktur im Einklang.

f) Herstellung von 2-Fluor-5,6,11,12-tetratellurtetracen

In einem 200 ml Sulfierkolben mit Rückflusskühler und Gaseinleitungsrohr sowie Thermometer werden unter einem leichten Argonfluss 205 g Tellur (16 mMol) und 368 mg Natrium (16 mMol) in 30 ml (über A4-Molekularsieb getrocknetem) DMF bei 110°C während 1 1/4 Stunden zum $Na_2Te_2$ (8 mMol) umgesetzt. Zu der auf 50°C abgekühlten Lösung wird eine Suspension von 1,4 g (3,66 mMol) 2-Fluor-5,6,11,12-tetrachlortetracen in 30 ml trockenem DMF gegeben und 92 Stunden bei 50°C gerührt. Die grüne Lösung wird unter Argon filtriert, der grauschwarze Niederschlag mit DMF, Benzol und Aceton gewaschen (je 3 x 25 ml Portionen). Das Rohprodukt, 3,23 g (>100 % enthält noch Kochsalz) wird aus 6 l Chlorbenzol unter einer Argonatmosphäre und in einem gebräunten Glaskolben umkristallisiert. Man erhält so 660 mg (24 %) reines 2-Fluor-5,6,11,12-tetratellurtetracen in Form schwarzer Nädelchen mit silbrigem Glanz.
UV-Vis-Spektrum: $\lambda_{max}$ (1,2,4-Trichlorbenzol) = 766, 708 (Schulter), 464 nm.
Massenspektrum: $M^+$ = 754 (4Te). Die Fragmentierung steht mit der erwarteten Struktur in Einklang.

g) Herstellung von

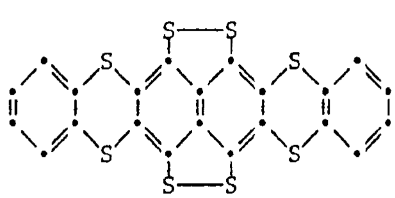

1,0 g (1,04 mMol) 1,2-Benzoldithiol, 0,78 g (13,93 mMol) KOH und 300 ml Dimethylacetamid (DMA) werden unter Argonatmosphäre zusammengegeben und zum Rückfluss erhitzt. Dann wird DMA abdestilliert, bis der Siedepunkt auf ca. 165°C gestiegen ist (ca. 100 ml). Man lässt etwas abkühlen, gibt 1,36 g (3,5 mMol) 2,3,6,7-Tetrachlor-1,4,5,8-tetrathionaphthalin zu, und erhitzt 30 Minuten am Rückfluss. Danach wird mit 400 ml Wasser verdünnt, der Niederschlag abgesaugt, mit Wasser, Ethanol und Chloroform gewaschen und am Hochvakuum getrocknet. Man erhält 1,55 g Rohprodukt (84 % der Theorie). Die Sublimation bei 360°C/0,13 Pascal ergibt 1,126 g braune Nadeln. Massenspektrum: $M^+ = 528$. $\lambda_{max}$ (1,2,4-Trichlorbenzol): 445 nm.

| Elementaranalyse: | % C | % H | % S |
|---|---|---|---|
| Berechnet: | 49,97 | 1,53 | 48,5 |
| Gefunden: | 50,09 | 1,45 | 48,8 |

h) Herstellung von

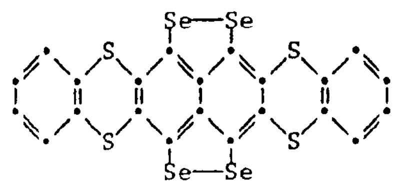

535 mg (3,76 mMol) 1,2-Benzoldithiol, 422 mg (7,53 mMol) KOH und 200 ml DMA werden unter Argonatmosphäre zusammengegeben und zum Rückfluss erhitzt. Es werden 50 ml DMA abdestilliert, leicht abgekühlt und 534 mg (0,92 mMol) 2,3,6,7-Tetrachlor-1,4,5,8-tetraselenonaphthalin zugegeben. Man hält 45 Minuten am Rückfluss. Nach dem Abkühlen wird der Niederschlag abgesaugt. Der Niederschlag wird mit DMA, Wasser, Ethanol und Chloroform gewaschen und im Hochvakuum getrocknet. Man erhält 288 mg Rohprodukt (43 % d.Th). 100 mg des Rohproduktes werden bei 360°C/0,13 Pascal sublimiert. Es ergeben sich 85 mg rostbraune Nadeln.
Massenspektrum: $M^+ = 720$ (Cluster 4Se). $\lambda_{max}$ (1,2,4-Trichlorbenzol): 460 nm.

i) Herstellung von

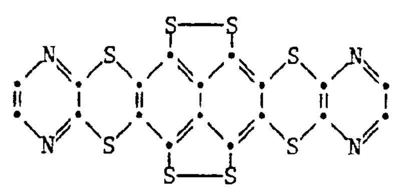

144 mg (1 mMol) Pyrazin-2,3-dithiol, 1,12 mg KOH (2 mMol) und 45 ml DMA werden unter Argonatmosphäre zusammengegeben und zum Rückfluss erhitzt. Es wird 30 Minuten am Rückluss gehalten und danach 15 ml DMA abdestilliert. Man lässt leicht abkühlen, gibt 194 mg (0,5 mMol) 2,3,6,7-Tetrachlor-1,4,5,8-tetrathionaphthalin zu und erhitzt 30 Minuten am Rückfluss. Nach dem Abkühlen wird der Niederschlag abgesaugt, mit DMA, Wasser und Ethanol gewaschen und im Hochvakuum getrocknet. Man erhält 14 mg Rohprodukt (5 % d.Th.). Die Sublimation bei 360°C/0,13 Pascal ergibt rostbraune Kristalle.
Massenspektrum: $M^+ = 532$. $\lambda_{max}$ (1,2,4-Trichlorbenzol): 435 nm.

j) Herstellung von 2,3,6,7-(Tetraphenylthio)-1,4,5,8-tetrathionaphthalin

220 mg (2 mMol) Thiophenol, 112 mg KOH und 50 ml DMA werden unter Argonatmosphäre zusammengegeben und zum Rückfluss erhitzt. Es werden 15 ml DMA abdestilliert. Man lässt leicht abkühlen, gibt 194 mg (0,5 mMol) 2,3,6,7-Tetrachlor-1,4,5,8-tetrathionaphthalin zu und hält eine Stunde am Rückfluss. Nach dem Abkühlen wird der braun-rote Niederschlag abgesaugt, mit DMA, Wasser, Ethanol und Chloroform gewaschen

und im Hochvakuum getrocknet. Man erhält 212 mg (61,9 % d.Th.) Rohprodukt. Die Sublimation bei 265°C/0,13 Pascal ergibt scharlachrote, durchsichtige Kristallstäbchen.
Schmelzpunkt: 285°C
Massenspektrum: $M^+ = 684$. $\lambda_{max}$ (1,2,4-Trichlorbenzol): 516 nm.

k) Herstellung von 2,3,6,7-Tetramethoxyphenyl-tetrathiotetracen (A) und 2,3,6,7-Tetra-4-pyridylthio-tetracen (B)

Wird unter sonst identischen Bedingungen wie im Herstellungsbeispiel j) an Stelle von Thiophenol 4-Methoxi-thiophenol bzw. 4-Mercaptopyridin verwendet so erhält man die Verbindungen A bzw. B.

| | UV-Vis-Spektrum [$\lambda_{max}$ (nm) (1,2,4-Trichlorbenzol)] | Massenspektrum [$M^+$] | Sublimationstemperatur (°C) |
|---|---|---|---|
| A | 508 | 804 | ~ 240 [schmilzt vor Sublimation] |
| B | 526 | 688 | ~ 300 |

l) Herstellung von 2,3,6,7-(Tetraphenylthio)-1,4,5,8-tetraselenonaphthalin

Unter identischen Bedingungen wie im Herstellungsbeispiel j) wird an Stelle von 2,3,6,7-Tetrachlor-1,4,5,8-tetrathionaphthalin 2,3,6,7-Tetrachlor-1,4,5,8-tetraselenonaphthalin mit Thiophenolat umgesetzt. Ausbeute: 33,2 %. Die Sublimation bei 310°C ($1,3 \cdot 10^{-3}$ mbar) ergibt ziegelrote Plättchen. $\lambda_{max}$ (1,2,4-Trichlorbenzol): 530 (Schulter), 469 nm; Massenspektrum: $M^+$: 872 (4Se). Die Fragmentierung steht mit der erwarteten Struktur im Einklang.

Anwendungsbeispiele

Beispiele 1-4:

Herstellung von elektrisch leitenden Polymerfilmen mit gasförmigem Elektronacceptor.

Ein Tetraselenotetracen und ein Polymer werden in einem Lösungsmittel unter Erwärmung gelöst. Die Lösung wird auf eine geheizte Glasplatte gegossen und das Lösungsmittel verdampft. Der Film wird bei der Abdampftemperatur mit einem gasförmigen Elektronenacceptor behandelt. Bevor der Film eine deutliche Rotfärbung aufweist, wird die Behandlung beendet. Dieser Zeitpunkt wird vorher durch Ausprobieren ermittelt. Weitere Angaben sind Tabelle 1 entnehmbar, wo auch der spezifische Widerstand angegeben ist.

Tabelle 1

| Beispiel Nr. | Polymer 1) | mg TSeT 2) | 9,9 g Lösungsmittel | Elektronenacceptor | Abdampftemperatur [°C] | spezifischer Widerstand [Ohm x cm] |
|---|---|---|---|---|---|---|
| 1 | PVC (Vinnol H70 DF/Wacker) | 1,6 2-FTSeT | Nitrobenzol | Chlor | 150 | 50 |
| 2 | Polyimid 3) | 2 TSeT | 1,2-Dichlorbenzol | Chlor | 140 | 14 |
| 3 | Polyimid 4) | 2 TSeT | 1,2-Dichlorbenzol | Luftsauerstoff | 120 | $7,8 \times 10^2$ |
| 4 | PVC (Vinnol H70 DF/Wacker) | 1,6 2,3,8,9-$F_4$TSeT | Nitrobenzol | Chlor | 150 | $4,1 \times 10^5$ |

1) 100 mg

2) Tetraselenotetracen

3) aus Pyromellitsäuredianhydrid und 2-Methyl-4,6-diethyl-1,5-diaminobenzol, $\eta$ = 0,5 dl/g (25°C, N-Methylpyrrolidon)

4) aus Pyromellitsäuredianhydrid, 2-Methyl-4,6-diethyl-1,5-diaminobenzol (60 Mol-%, bezogen auf Diamine) und 2,2'-Dimethyl-4,4'-diaminodiphenylmethan (40 Mol-%), $\eta$ = 0,92 dl/g (25°C, N-Methylpyrrolidon)

Beispiele 5-25:

Herstellung von elektrisch leitenden Polymerfilmen mit einer flüssigen oder festen Halogen bildenden Verbindung.

100 mg Polymer (200 mg in Beispiel 10) und ein Tetraselenotetracen werden in einem Lösungsmittel I gelöst. Danach versetzt man die Lösung mit einer Halogen bildenden Verbindung, die ihrerseits in einem Lösungsmittel II gelöst ist (Beispiele 5, 8, 9, 12, 13, 18-22, 25). Die Lösung wird auf eine beheizte Glasplatte gegossen und das Lösungsmittel bei erhöhter Temperatur verdampft. Danach wird der spezifische Widerstand bestimmt. Weitere Angaben sind der Tabelle 2 entnehmbar.

Tabelle 2

| Beispiel Nr. | Polymer | Menge TSeT 2) [mg] | Lösungsmittel I [Menge in g] | Halogen bildende Verbindung | Lösungsmittel II [Menge in ml] | Abdampftemperatur [°C] | spezifischer Widerstand [Ohm x cm] |
|---|---|---|---|---|---|---|---|
| 5 | Polysulfon 5) Resin (Polyscience) | 2,3-$F_2$TSeT [1.6] | Nitrobenzol [6,3] | Tetrabrommethan [6,0 mg] | Nitrobenzol [3,0] | 130 | 0,97 |
| 6 | PVC (Vinnol H70 DF, Wacker) | 2-FTSeT [1.6] | 1,2-Dichlorbenzol [8,9] | Bromoform [5,8 x $10^3$ mg] | -- | 130 | 12,6 |
| 7 | Polysulfon 6) (Victrex 4800 P) | TSeT [1,6] | Nitrobenzol [7,5] | Bromoform [5,8 x $10^3$ mg] | -- | 120 | 0,2 |
| 8 | Polysulfon 6) (Victrex 4800 P) | 2-FTSeT [1,7] | Nitrobenzol [7,5] | o-Chloranil [0,7 mg] | 1,2-Dichlorbenzol [1,0] | 130 | 122 |
| 9 | Polysulfon 5) Resin | 2-FTSeT [1,6] | Dimethylformamid [6,0] | Hexachlorpropan [4,2 mg] | Dimethylformamid [2,0] | 120 | 0,56 |
| 10 | Polyimid 3) | 2-FTSeT [1,6] | Nitrobenzol [4,0] | Bromoform [5,8 x $10^3$ mg] | -- | 130 | 9,0 |
| 11 | Polyimid 4) | TSeT [1,6] | Nitrobenzol [9,9] | Bromoform [5,8 x $10^3$ mg] | -- | 160 | 0,44 |
| 12 | Polysulfon 5) Resin | TSeT [1,6] | 1,2,4-Trichlorbenzol [7,5] | Hexachlorpropen [1,9 mg] | 1,2,4-Trichlorbenzol [2,0] | 160 | 0,17 |
| 13 | Polysulfon 5) Resin | TSeT [1,6] | γ-Butyrolacton [7,5] | Hexachlorpropen [1,9 mg] | γ-Butyrolacton [2,0] | 130 | 2,1 |
| 14 | Polyamid 4) | TSeT [1,6] | DMF [9,8] | Perchlorbutadien [5 µl] | - | 100 | 2,6 |

Tabelle 2 (Fortsetzung)

| Beispiel Nr. | Polymer | Menge TSeT 2) [mg] | Lösungsmittel I [Menge in g] | Halogen bildende Verbindung | Lösungsmittel II [Menge in ml] | Abdampftemperatur [°C] | spezifischer Widerstand [Ohm x cm] |
|---|---|---|---|---|---|---|---|
| 15 | Polyimid 4) | TSeT [1,6] | DMF [9,8] | Dichloracetaldehyddiethylacetal [5 µl] | - | 90 | 7,6 |
| 16 | Polimid 4) | TSeT [1,6] | DMF [9,8] | 1,4-Dichlor-2-buten [5 µl] | - | 120 | 2,7 |
| 17 | Polyimid 4) | TSeT [1,6] | DMF [9,8] | 1,3-Dichlor-2-buten [5 µl] | - | 110 | 0,48 |
| 18 | Polyimid 4) | TSeT [1,6] | DMF [9,8] | 1,3-Dichloraceton [5 mg] | DMF [2,0] | 130 | 13,7 |
| 19 | Polyimid 4) | TSeT [1,6] | DMF [9,8] | α,α',2,3,5,6-Hexachlor-p-xylol [5 mg] | DMF [2,0] | 90 | 0,80 |
| 20 | Polyimid 4) | TSeT [1,6] | DMF [9,8] | 1,4-Bis(trichloromethyl)-benzol [5 mg] | DMF [2,0] | 130 | 4,9 |
| 21 | Polycarbonat 9) | TSeT [1,6] | 1,2,4-Trichlorbenzol [9,0] | Hexachlorpropen [5,1 mg] | 1,2,4-Trichlorbenzol [2,0] | 170 | 0,25 |
| 22 | Polycarbonat 9) | TSeT [1,6] | Chlorbenzol [7,0] | Hexachlorpropen [5,2 mg] | Chlorbenzol [1,6] | 120 | 472 |
| 23 | Polyimid 3) | TSeT [1,6] | DMF [9,8] | Jodacetonitril [5 µl] | - | 130 | 1,2 |

Tabelle 2 (Fortsetzung)

| Beispiel Nr. | Polymer | Menge TSeT 2) [mg] | Lösungsmittel I [Menge in g] | Halogen bildende Verbindung | Lösungsmittel II [Menge in ml] | Abdampftemperatur [°C] | spezifischer Widerstand [Ohm x cm] |
|---|---|---|---|---|---|---|---|
| 24 | Polycarbonat 9) | TTT [1,6] 7) | 1,2-Dichlorbenzol [9,9] | Diiodmethan [0,4 ml] | - | 140 | 30 |
| 25 | Polycarbonat 9) | TPT-TTN [1,6] 8) | Dichlormethan [10,3] | Brom [5,56 mg] | Dichlormethan [1,0] | 30 | $2{,}9 \cdot 10^5$ |

2) wie Tabelle 1

3) wie Tabelle 1

4) wie Tabelle 1

5) $-\!\!\left(O-C_6H_4-C(CH_3)_2-C_6H_4-O-C_6H_4-SO_2-C_6H_4\right)_n\!\!-$

6) $-\!\!\left(C_6H_4-O-C_6H_4-SO_2\right)_n\!\!-$

7) Tetrathiotetracen

8) 2,3,6,7-Tetraphenylthio-1,4,5,8-tetrathionaphthalin

9) Bisphenol-A-polycarbonat (PC 3000 W)

Beispiel 26:

1,6 mg TSeT, 1,6 mg 2,3,6,7-(Tetraphenylthio)-1,4,5,8-tetrathionaphthalin und 100 mg Polycarbonat werden in 9,0 g 1,2,4-Trichlorbenzol gelöst. Zur Lösung werden 5,1 mg Hexachlorpropen in 2,0 ml 1,2,4-Trichlorbenzol gegeben. Mit 1,3 ml der Lösung wird eine Folie gegossen. Das Lösungsmittel wird bei 150°C abgedampft. Es wird eine Folie mit einem spezifischen Widerstand von 0,15 Ωcm und ausgeprägten metallischen Eigenschaften erhalten. Der alleinige Zusatz von 2,3,6,7-(Tetraphenylthio)-1,4,5,8-tetrathionaphthalin ergibt Folien mit wesentlich höherem spezifischem Widerstand (siehe Beispiel 25). Auch Folien mit TSeT als alleinigem Zusatz weisen noch einen höheren spezifischen Widerstand auf (siehe Beispiel 21).

Beispiele 27-75:

100 mg Polymer und 1,6 mg Tetraselenotetracen werden in einem Lösungsmittel gelöst. Dann versetzt man diese Lösung mit der Lösung einer halogenhaltigen Verbindung als Elektronenacceptor. Die Lösung wird auf eine beheizte Glasplatte gegossen; unter isothermen Bedingungen lässt man das Lösungsmittel bei Temperaturen zwischen 100-130°C abdampfen. Tabelle 3 zeigt die geprüften Verbindungen. In allen Fällen werden leitende Polymerfolien mit einem elektrisch leitenden Netzwerk aus Kristallnadeln erhalten. Die Leitfähigkeit beträgt zwischen 0,1 bis 5 $Scm^{-1}$.

Tabelle 3:

| Beispiel-Nr. | Polymer | Halogenverbindung | Lösungsmittel |
|---|---|---|---|
| 27 | PI | 1,12-Dibrom-dodecan | DMF |
| 28 | PI | α,α'-Dibrom-p-xylol | DMF |
| 29 | PI | Phenacylchlorid | DMF |
| 30 | PI | α,α'-Dichlor-o-xylol | DMF |
| 31 | PI | Phenacylbromid | DMF |
| 32 | PI | 1,10-Dibromdecan | DMF |

Tabelle 3: (Fortsetzung)

| Beispiel-Nr. | Polymer | Halogenverbindung | Lösungsmittel |
|---|---|---|---|
| 33 | PI | α,α'-Dichlor-p-xylol | DMF |
| 34 | PI | α,α'-Dibrom-m-xylol | DMF |
| 35 | PI | Jodacetonitril | DMF |
| 36 | PI | 2,3-Dichlorpropionsäure-methylester | DMF |
| 37 | Polyarylat | Hexachlorpropen | DMF |
| 38 | PI | Hexachlorpropen, 1,1,2-Trichlor-ethan 1:1 | DMF |
| 39 | PI | Hexachlorpropen, 1,6-Dibromhexan 1:1 | DMF |
| 40 | PI | Hexachlorpropen, Jodacetonitril 1:1 | DMF |
| 41 | PI | 1,6-Dibromhexan, Jodacetonitril 1:1 | DMF |
| 42 | PI | Jodacetonitril | DMF |
| 43 | PI | 1-Brom-2-Chlorethan | DMF |
| 44 | PI | 1-Brom-2-Chlor-propan | DMF |
| 45 | PI | Chlorameisensäure-2-Bromethylester | DMF |
| 46 | PI | Jodessigsäure-ethylester | DMF |
| 47 | PI | N-Bromsuccinimid | DMF |
| 48 | PI | N-Chlorsuccinimid | DMF |
| 49 | PI | N-Jodsuccinimid | DMF |
| 50 | PC | Hexachloraceton | TCB |
| 51 | PC | 1,4,5,6,7,7-Hexachlor-5-norbornen-2,3-dicarbonsäure anhydrid | TCB |
| 52 | PC | 1,2,5,6,9,10-Hexabrom-cyclododecan | TCB |
| 53 | PI | Tetrachlorethylen | DMF |
| 54 | PI | Perchlorcyclopentadien | DMF |
| 55 | PI | Perchlorbutadien | DMF |
| 56 | PI | Dichloracetaldehyd-diethylacetal | DMF |
| 57 | PI | 1,4-Dichlor-2-buten | DMF |

Tabelle 3: (Fortsetzung)

| Beispiel-Nr. | Polymer | Halogenverbindung | Lösungsmittel |
|---|---|---|---|
| 58 | PI | 1,3-Dichlor-2-buten | DMF |
| 59 | PI | 3,4-Dichlor-1-buten | DMF |
| 60 | PI | Tetrachlorcyclopropen | DMF |
| 61 | PI | 1,3-Dichloraceton | DMF |
| 62 | PI | 2,3,5,6-Hexachlor-p-xylol | DMF |
| 63 | PI | 1,4-Bis(trichloromethyl)-benzol | DMF |
| 64 | PI | 1,3-Dibrom-propan | DMF |
| 65 | PI | 1,6-Dibrom-hexan | DMF |
| 66 | PI | 3-Chlor-propionsäure-ethylester | DMF |
| 67 | PI | 3-Chlor-toluol | DMF |
| 68 | PI | 2-Chlor-propionsäure-methylester | DMF |
| 69 | PI | 2-Chlor-acrylonitril | DMF |
| 70 | PI | Trichloressigsäure-ethylester | DMF |
| 71 | PI | 1,2,3-Trichlor-propan | DMF |
| 72 | PI | 1,1,2-Trichlor-ethan | DMF |
| 73 | PI | Chlorameisensäure-butylester | DMF |
| 74 | PI | Trichlorethylen | DMF |
| 75 | PI | 2,3-Dichlormaleinsäureanhydrid | DMF |

PI : Polyimid gemäss Beispiel 2
PC : Bisphenol-A-Polycarbonat (PC 3000 W)
Polyarylat: Polyester aus Terephthalsäure und Bisphenol A
DMF : Dimethylformamid
TCB : 1,2,4-Trichlorbenzol

Beispiele 76 und 77:

100 mg Polyimid und 1,6 g Tetraselenotetracen werden in 10 g Dimethylformamid gelöst. Dann versetzt man diese Lösung mit 0,5 Aequivalenten der Elektronenacceptoren Eisen(III)chlorid bzw. Nitronium-tetrafluoroborat. Die Lösung wird auf eine beheizte Glasplatte gegossen, unter isothermen Bedingungen lässt man das Lösungsmittel bei Temperaturen zwischen 100-130°C abdampfen. Man erhält leitende Polymerfolien mit einem elektrisch leitenden Netzwerk aus Kristallnadeln; die Leitfähigkeit gemessen nach der 4-Punktmethode beträgt 0,01-0,5 $Scm^{-1}$.

**Patentansprüche**

**Patentansprüche für folgende Vertragsstaaten: AT, BE, CH, DE, FR, GB, IT, LI, NL, SE**

1. Zusammensetzung, enthaltend
a) ein lineares, verzweigtes oder strukturell vernetztes Polymer, das gegenüber Komponente b) inert ist, und
b) eine Verbindung der Formel I oder Ia oder Mischungen davon,

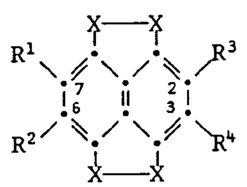 (I), 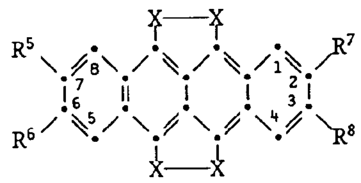 (Ia),

worin X für S, Se oder Te steht, $R^1$, $R^2$, $R^3$ und $R^4$ unabhängig voneinander ein Wasserstoffatom oder Cl bedeuten oder $R^1$ und $R^2$ sowie $R^3$ und $R^4$ zusammen je

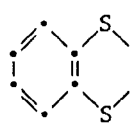 oder 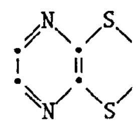 oder

$R^1$, $R^2$, $R^3$ und $R^4$ je Phenylthio, 4-Methyl- oder 4-Methoxyphenylthio oder 4-Pyridylthio darstellen, $R^5$, $R^6$, $R^7$ und $R^8$ unabhängig voneinander H oder F bedeuten, $R^5$ für $CH_3$ und $R^6$, $R^7$ und $R^8$ für H oder $R^5$, $R^6$, $R^7$ und $R^8$ für $CH_3$ stehen, $R^5$ und $R^6$ für $CH_3$ oder Cl und $R^7$ und $R^8$ für H stehen oder $R^5$ und $R^6$ für H, $R^7$ für $-COR^9$ und $R^8$ für H oder $-COR^9$, oder $R^5$ und $R^6$ für H und $R^7$ und $R^8$ zusammen für -CO-O-CO oder $-CO-NR^{10}-CO-$ stehen, worin $R^9$ Halogenid, -OH, $-NH_2$, $C_1$-$C_6$-Alkoxy, $C_2$-$C_6$-Hydroxyalkoxy, Benzyloxy, Phenoxy, Cyclopentyloxy, Cyclohexyloxy, $C_1$-$C_6$-Alkylamino oder Di($C_1$-$C_6$-Alkyl)amino darstellt, oder -OM ist, wobei M ein Metall- oder Ammoniumkation bedeutet, und $R^{10}$ H, $C_1$-$C_{18}$-Alkyl, Phenyl oder Benzyl ist.

2. Zusammensetzung gemäss Anspruch 1, dadurch gekennzeichnet, dass die Komponente b) in einer Menge von 0,01 bis 20 Gew.-% enthalten ist, bezogen auf das Polymer.

3. Zusammensetzung gemäss Anspruch 1, dadurch gekennzeichnet, dass es sich bei Komponente b) um Tetrathiotetracen, Tetraselenotetracen, 2-Fluor- oder 2,3-Difluortetraselenotetracen handelt.

4. Zusammensetzung gemäss Anspruch 1, dadurch gekennzeichnet, dass sie eine Mischung von Verbindungen der Formeln I und Ia enthält.

5. Zusammensetzung gemäss Anspruch 1, dadurch gekennzeichnet, dass zusätzlich ein Lösungsmittel für ein lösliches Polymer und die Komponente b) enthalten ist.

6. Zusammensetzung gemäss Anspruch 1, dadurch gekennzeichnet, dass sie zusätzlich einen Elektronenacceptor enthält.

7. Zusammensetzung gemäss Anspruch 6, dadurch gekennzeichnet, dass es sich bei dem Elektronenacceptor um eine halogenhaltige, organische Verbindung handelt, die unter Zufuhr von Energie Halogen abspaltet.

8. Zusammensetzung gemäss Anspruch 6, dadurch gekennzeichnet, dass das Mengenverhältnis von Elektronenacceptor zu Komponente b) 10:1 bis 1:5 beträgt.

9. Zusammensetzung gemäss Anspruch 7, dadurch gekennzeichnet, dass es sich bei der halogenhaltigen Verbindung um eine halogenierte, gesättigte oder ungesättigte, aliphatische, cycloaliphatische, aliphatisch-heterocyclische, aromatische oder heteroaromatische organische Verbindung handelt.

10. Zusammensetzung gemäss Anspruch 9, dadurch gekennzeichnet, dass die organische Verbindung chloriert, bromiert und/oder jodiert ist.

11. Zusammensetzung gemäss Anspruch 7, dadurch gekennzeichnet, dass es sich bei der halogenhaltigen organischen Verbindung um Tetrabrommethan, Bromoform, Trichlorbrommethan, Hexachlorpropen, Hexachlorcyclopropan, Hexachlorcyclopentadien, Hexachlorethan, N-Chlorsuccinimid, Octachlorpropan, n-Octachlorbutan, n-Decachlorbutan, Tetrabromethan, Hexabromethan, Tetrabromo-o-benzochinon, N-Bromsuccinimid, 2,4,4,6-Tetrabromo-2,5-cyclohexadienon, Hexabrombenzol, Chloranil, Hexachloraceton,

1,4,5,6,7,7-Hexachlor-5-norbornen-2,3-dicarbonsäure, 1,2,5,6,9,10-Hexabromcyclododecan, Tetrachlorethylen, Perchlorcyclopentadien, Perchlorbutadien, Dichloracetaldehyd-diethylacetal, 1,4-Dichlor-2-buten, 1,3-Dichlor-2-buten, 3,4-Dichlor-1-buten, Tetrachlorcyclopropen, 1,3-Dichloraceton, 2,3,5,6-Hexachlor-p-xylol, 1,4-Bis(trichlormethyl)-benzol, 1,3-Dibrompropan, 1,6-Dibromhexan, 3-Chlorpropionsäureethylester, 3-Chlortoluol, 2-Chlorpropionsäuremethylester, 2-Chloracrylnitril, Trichloressigsäureethylester, 1,2,3-Trichlorpropan, 1,1,2-Trichlorethan, Chlorameisensäurebutylester, Trichlorethylen, 2,3-Dichlormaleinsäureanhydrid, 1,12-Dibromdodecan, $\alpha,\alpha'$-Dibrom-p-xylol, $\alpha,\alpha'$-Dichlor-o-xylol, Phenacylchlorid oder -bromid, 1,10-Dibromdecan, $\alpha,\alpha'$-Dichlor-p-xylol, $\alpha,\alpha'$-Dibrom-m-xylol, Jodacetonitril, 2,3-Dichlor-5,6-dicyanobenzochinon, 2,3-Dichlorpropionsäuremethylester, 1-Brom-2-chlorethan, 1-Brom-2-chlorpropan, Chlorameisensäure-2-bromethylester, Jodessigsäureethylester, N-Chlor-, N-Brom- oder N-Jodsuccinimid oder -phthalsäureimid oder Mischungen hiervon handelt.

12. Zusammensetzung gemäss Anspruch 7, dadurch gekennzeichnet, dass die halogenhaltige organische Verbindung flüssig ist und gleichzeitig ein Lösungsmittel für das Polymer und die Komponente b) ist.

13. Zusammensetzung gemäss Anspruch 1, dadurch gekennzeichnet, dass es sich bei dem Polymer um Duroplaste, Thermoplaste oder Elastomere handelt.

14. Zusammensetzung enthaltend

a) ein lineares, verzweigtes oder strukturell vernetztes Polymer und

b) ein Netzwerk aus elektrisch leitenden Kristallnadeln in der Polymermatrix von mindestens einem Charge-Transfer-Komplex (CT-Komplex) aus mindestens einer Verbindung der Formeln I oder Ia gemäss Anspruch 1 in form von Chloriden, Bromiden oder Iodiden, oder mit $O_2$ oder einem Salz eines oxidativ wirkenden Kations mit nicht nukleophilen Anionen als Elektronenakzeptor.

15. Zusammensetzung gemäss Anspruch 14, dadurch gekennzeichnet, dass der CT-Komplex in einer Menge von 0,01 bis 20 Gew.-% enthalten ist, bezogen auf das Polymer.

16. Verfahren zur Herstellung einer Zusammensetzung gemäss Anspruch 14, bei dem man auf eine Zusammensetzung gemäss Anspruch 1 $O_2$, ein gas- oder dampfförmiges Halogenierungsmittel, eine organische halogenhaltige Verbindung, welche bei Energiezufuhr Halogen bildet, oder ein Salz eines oxidativ wirkenden Kations mit nicht nukleophilen Anionen als Elektronenacceptor einwirken lässt.

17. Verfahren gemäss Anspruch 16, dadurch gekennzeichnet, dass der Elektronenacceptor Chlor, Brom und/oder Iod ist, die durch Erwärmen einer in der Zusammensetzung enthaltenen Cl, Br und/oder I bildenden organischen Verbindung freigesetzt werden.

18. Geformte Gegenstände, Beschichtungen und Verbundstoffe aus einer Zusammensetzung gemäss Anspruch 14.

19. Verbindungen der Formel II oder IIa

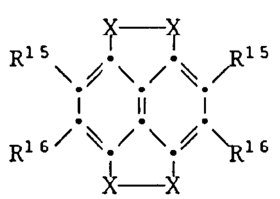 (II), 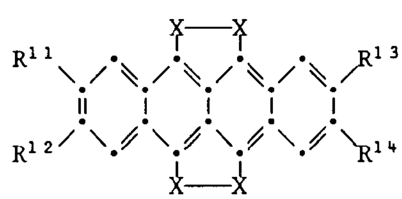 (IIa),

worin $R^{15}$ und $R^{16}$ je Phenylthio, 4-Methyl- oder 4-Methoxyphenylthio oder 4-Pyridylthio oder worin $R^{15}$ und $R^{16}$ zusammen

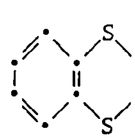 oder 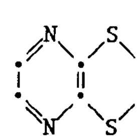

bedeuten, $R^{11}$ für $-CH_3$ und $R^{12}$, $R^{13}$ und $R^{14}$ für H, $R^{11}$ und $R^{12}$ für Cl oder $CH_3$ und $R^{13}$ und $R^{14}$ für H, oder $R^{11}$, $R^{12}$, $R^{13}$ und $R^{14}$ für $CH_3$ oder F stehen, und X S, Se oder Te darstellt.

**Patentansprüche für fogende Vertragsstaaten: ES, GR**

1. Verfahren zur Herstellung einer Zusammensetzung enthaltend

a) ein lineares, verzweigtes oder strukturell vernetztes Polymer und

b) mindestens einen Charge-Transfer-Komplex (CT-Komplex), der aus mindestens einer Verbindung der Formeln I oder Ia und einem Elektronenacceptor gebildet ist,

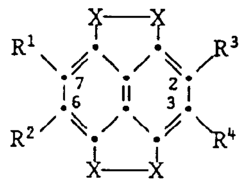 (I), 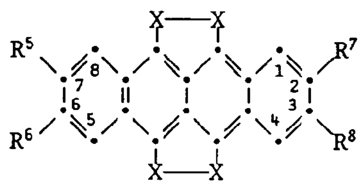 (Ia),

worin X für S, Se oder Te steht, $R^1$, $R^2$, $R^3$ und $R^4$ unabhängig voneinander ein Wasserstoffatom oder Cl bedeuten oder $R^1$ und $R^2$ sowie $R^3$ und $R^4$ zusammen je

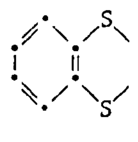 oder 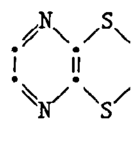 oder

$R^1$, $R^2$, $R^3$ und $R^4$ je Phenylthio, 4-Methyl- oder 4-Methoxyphenylthio oder 4-Pyridylthio darstellen, $R^5$, $R^6$, $R^7$ und $R^8$ unabhängig voneinander H oder F bedeuten, $R^5$ für $CH_3$ und $R^6$, $R^7$ und $R^8$ für H oder $R^5$, $R^6$, $R^7$ und $R^8$ für $CH_3$ stehen, $R^5$ und $R^6$ für $CH_3$ oder Cl und $R^7$ und $R^8$ für H stehen oder $R^5$ und $R^6$ für H, $R^7$ für -$COR^9$ und $R^8$ für H oder -$COR^9$, oder $R^5$ und $R^6$ für H und $R^7$ und $R^8$ zusammen für -CO-O-CO oder -CO-$NR^{10}$-CO- stehen, worin $R^9$ Halogenid, -OH, -$NH_2$, $C_1$-$C_6$-Alkoxy, $C_2$-$C_6$-Hydroxyalkoxy, Benzyloxy, Phenoxy, Cyclopentyloxy, Cyclohexyloxy, $C_1$-$C_6$-Alkylamino oder Di($C_1$-$C_6$-Alkyl)amino darstellt, oder -OM ist, wobei M ein Metall- oder Ammoniumkation bedeutet, und $R^{10}$ H, $C_1$-$C_{18}$-Alkyl, Phenyl oder Benzyl ist, dadurch gekennzeichnet, dass man auf eine Zusammensetzung, enthaltend

a) ein lineares, verzweigtes oder strukturell vernetztes Polymer, das gegenüber Komponente b) inert ist, und

b) eine Verbindung der Formel I oder Ia oder Mischungen davon, $O_2$, ein gas- oder dampfförmiges Halogenierungsmittel, eine organische halogenhaltige Verbindung, welche bei Energiezufuhr Halogen bildet, oder ein Salz eines oxidativ wirkenden Kations mit nicht nukleophilen Anionen,

als Elektronenacceptor einwirken lässt.

2. Verfahren gemäss Anspruch 1, dadurch gekennzeichnet, dass der CT-Komplex in einer Menge von 0,01 bis 20 Gew.-% enthalten ist, bezogen auf das Polymer.

3. Verfahren gemäss Anspruch 1, dadurch gekennzeichnet, dass es sich bei Komponente b) um Tetrathiotetracen, Tetraselenotetracen, 2-Fluor- oder 2,3-Difluortetraselenotetracen handelt.

4. Verfahren gemäss Anspruch 1, dadurch gekennzeichnet, dass es sich bei der Komponente b) um eine Mischung von Verbindungen der Formeln I und Ia handelt.

5. Verfahren gemäss Anspruch 1, dadurch gekennzeichnet, dass die Zusammensetzung zusätzlich ein Lösungsmittel für ein lösliches und inertes Polymer und die Komponente b) enthält.

6. Verfahren gemäss Anspruch 1, dadurch gekennzeichnet, dass es sich bei dem Elektronenacceptor um eine halogenhaltige, organische Verbindung handelt, die unter Zufuhr von Energie Halogen abspaltet.

7. Verfahren gemäss Anspruch 6, dadurch gekennzeichnet, dass der Elektronenacceptor Chlor, Brom und/oder Iod ist, die durch Erwärmen einer in der Zusammensetzung enthaltenen Cl, Br und/oder I bildenden organischen Verbindungen freigesetzt werden.

8. Verfahren gemäss Anspruch 1, dadurch gekennzeichnet, dass das Mengenverhältnis von Elektronenacceptor zu Komponente b) 10:1 bis 1:5 beträgt.

9. Verfahren gemäss Anspruch 7, dadurch gekennzeichnet, dass es sich bei der halogenhaltigen Verbindung um eine halogenierte, gesättigte oder ungesättigte, aliphatische, cycloaliphatische, aliphatisch-heterocyclische, aromatische oder heteroaromatische organische Verbindung handelt.

10. Verfahren gemäss Anspruch 6, dadurch gekennzeichnet, dass es sich bei der halogenhaltigen organischen Verbindungen um Tetrabrommethan, Bromoform, Trichlorbrommethan, Hexachlorpropen, Hexachlorcyclopropan, Hexachlorcyclopentadien, Hexachlorethan, N-Chlorsuccinimid, Octachlorpropan, n-Octachlorbutan, n-Decachlorbutan, Tetrabromethan, Hexabromethan, Tetrabromo-o-benzochinon, N-Bromsuccinimid, 2,4,4,6-Tetrabromo-2,5-cyclohexadienon, Hexabrombenzol, Chloranil, Hexachloraceton, 1,4,5,6,7,7-Hexachlor-5-norbornen-2,3-dicarbonsäure, 1,2,5,6,9,10-Hexabromcyclododecan, Tetrachlorethylen, Perchlorcyclopentadien, Perchlorbutadien, Dichloracetaldehyd-diethylacetal, 1,4-Dichlor-2-buten, 1,3-Dichlor-2-buten, 3,4-Dichlor-1-buten, Tetrachlorcyclopropen, 1,3-Dichloraceton, 2,3,5,6-Hexachlor-p-xylol, 1,4-Bis(trichlormethyl)-benzol, 1,3-Dibrompropan, 1,6-Dibromhexan, 3-Chlorpropionsäureethylester, 3-Chlortoluol, 2-Chlorpropionsäuremethylester, 2-Chloracrylnitril, Trichloressigsäureethylester, 1,2,3-Trichlorpropan, 1,1,2-Trichlorethan, Chlorameisensäurebutylester, Trichlorethylen, 2,3-Dichlormaleinsäureanhydrid, 1,12-Dibromdodecan, α,α'-Dibrom-p-xylol, α,α'-Dichlor-o-xylol, Phenacylchlorid oder -bromid, 1,10-Dibromdecan, α,α'-Dichlor-p-xylol, α,α'-Dibrom-m-xylol, Jodacetonitril, 2,3-Dichlor-5,6-dicyanobenzochinon, 2,3-Dichlorpropionsäuremethylester, 1-Brom-2-chlorethan, 1-Brom-2-chlorpropan, Chlorameisensäure-2-bromethylester, Jodessigsäureethylester, N-Chlor-, N-Brom- oder N-Jodsuccinimid oder -phthalsäureimid oder Mischungen hiervon handelt.

11. Verfahren gemäss Anspruch 6, dadurch gekennzeichnet, dass die halogenhaltige organische Verbindung flüssig ist und gleichzeitig ein Lösungsmittel für das inerte Polymer und die Komponente b) ist.

12. Verfahren gemäss Anspruch 1, dadurch gekennzeichnet, dass es sich bei dem Polymer um Duroplaste, Thermoplaste oder Elastomere handelt.

## Claims

### Claims for the following Contracting States: AT, BE, CH, DE, FR, GB, IT, LI, NL, SE

1. A composition containing
a) a linear, branched or structurally crosslinked polymer which is inert towards component b), and
b) a compound of the formula I or Ia or mixtures thereof

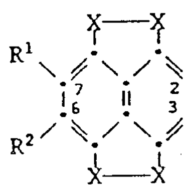
(I)

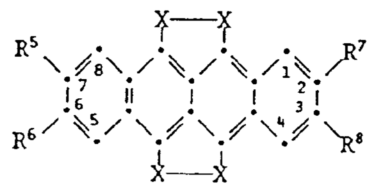
(Ia)

in which X is S, Se or Te, $R^1$, $R^2$, $R^3$ and $R^4$ independently of one another are a hydrogen atom or Cl or $R^1$ and $R^2$ and also $R^3$ and $R^4$ together are each

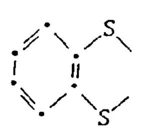
or
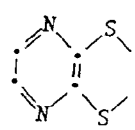

or $R^1$, $R^2$, $R^3$ and $R^4$ are each phenylthio, 4-methylphenylthio, 4-methoxyphenylthio or 4-pyridylthio, $R^5$, $R^6$, $R^7$ and $R^8$ independently of one another are H or F, $R^5$ is $CH_3$ and $R^6$, $R^7$ and $R^8$ are H, or $R^5$, $R^6$, $R^7$ and $R^8$ are $CH_3$, $R^5$ and $R^6$ are $CH_3$ or Cl, and $R^7$ and $R^8$ are H, or $R^5$ and $R^6$ are H, $R^7$ is $-COR^9$ and $R^8$ is H or $-COR^9$, or $R^5$ and $R^6$ are H and $R^7$ and $R^8$ together are -CO-O-CO or $-CO-NR^{10}-CO-$ in which $R^9$ is halide, -OH, $-NH_2$, $C_1$-$C_6$alkoxy, $C_2$-$C_6$hydroxyalkoxy, benzyloxy, phenoxy, cyclopentyloxy, cyclohexyloxy, $C_1$-$C_6$alkylamino or di-($C_1$-$C_6$alkyl)amino or is -OM in which M is a metal or ammonium cation, and $R^{10}$ is H, $C_1$-$C_{18}$alkyl, phenyl or benzyl.

2. A composition according to claim 1, wherein the component b) is present in an amount of 0.01 to 20% by weight, relative to the polymer.

3. A composition according to claim 1, wherein the component b) is tetrathiotetracene, tetraselenotetracene, 2-fluorotetraselenotetracene or 2,3-difluorotetraselenotetracene.

4. A composition according to claim 1, which contains a mixture of compounds of the formulae I and Ia.

5. A composition according to claim 1, which contains, in addition, a solvent for a soluble polymer and the component b).

6. A composition according to claim 1, which contains, in addition, an electron acceptor.

7. A composition according to claim 6, wherein the electron acceptor is a halogen-containing, organic compound which, when energy is supplied, splits off halogen.

8. A composition according to claim 6, wherein the ratio of the electron acceptor to the component b) is

10:1 to 1:5.

9. A composition according to claim 7, wherein the halogen-containing compound is a halogenated, saturated or unsaturated, aliphatic, cycloaliphatic, aliphatic-heterocyclic, aromatic or heteroaromatic, organic compound.

10. A composition according to claim 9, wherein the organic compound is chlorinated, brominated and/or iodinated.

11. A composition according to claim 7, wherein the halogen-containing, organic compound is tetrabromomethane, bromoform, trichlorobromomethane, hexachloropropene, hexachlorocyclopropane, hexachlorocyclopentadiene, hexachloroethane, N-chlorosuccinimide, octachloropropane, n-octachlorobutane, n-decachlorobutane, tetrabromoethane, hexabromoethane, tetrabromo-o-benzoquinone, N-bromosuccinimide, 2,4,4,6-tetrabromo-2,5-cyclohexadienone, hexabromobenzene, chloranil, hexachloroacetone, 1,4,5,6,7,7-hexachloro-5-norbornene-2,3-dicarboxylic acid, 1,2,5,6,9,10-hexabromocyclododecane, tetrachloroethylene, perchlorocyclopentadiene, perchlorobutadiene, dichloroacetaldehyde diethylacetal, 1,4-dichloro-2-butene, 1,3-dichloro-2-butene, 3,4-dichloro-1-butene, tetrachlorocylcopropene, 1,3-dichloroacetone, 2,3,5,6-hexachloro-p-xylene, 1,4-bis-(trichloromethyl)-benzene, 1,3-dibromopropane, 1,6-dibromohexane, ethyl 3-chloropropionate, 3-chlorotoluene, methyl 2-chloropropionate, 2-chloroacrylonitrile, ethyl trichloroacetate, 1,2,3-trichloropropane, 1,1,2-trichloroethane, butyl chloroformate, trichloroethylene, 2,3-dichloromaleic anhydride, 1,12-dibromododecane, $\alpha,\alpha'$-dibromo-p-xylene, $\alpha,\alpha'$-dichloro-o-xylene, phenacyl chloride or bromide, 1,10-dibromodecane, $\alpha,\alpha'$-dichloro-p-xylene, $\alpha,\alpha'$-dibromo-m-xylene, iodoacetonitrile, 2,3-dichloro-5,6-dicyanobenzoquinone, methyl 2,3-dichloropropionate, 1-bromo-2-chloroethane, 1-bromo-2-chloropropane, 2-bromoethyl chloroformate, ethyl iodoacetate, N-chlorosuccinimide, N-bromosuccinimide, N-iodosuccinimide, N-chlorophthalimide, N-bromophthalimide or N-iodophthalimide or mixtures thereof.

12. A composition according to claim 7, wherein the halogen-containing, organic compound is liquid and is at the same time a solvent for the polymer and the component b).

13. A composition according to claim 1, wherein the polymer is a thermosetting plastic, a thermoplastic or an elastomer.

14. A composition containing

a) a linear, branched or structurally cross-linked polymer and

b) a network of electrically conducting crystal needles in the polymer matrix of at least one charge-transfer complex (CT complex) composed of at least one compound of the formula I or Ia according to claim 1 in the form of chlorides, bromides or iodides or with $O_2$ or a salt of a cation having an oxidative action with non-nucleophilic anions as electron acceptor.

15. A composition according to claim 14, wherein the CT complex is present in an amount of 0.01 to 20% by weight, relative to the polymer.

16. A process for the preparation of a composition according to claim 14, in which $O_2$, a halogenating agent in the form of gas or vapour, an organic, halogen-containing compound which forms halogen when energy is supplied, or a salt of a cation having an oxidative action with non-nucleophilic anions is allowed to act as an electron acceptor on a composition according to claim 1.

17. A process according to claim 16, wherein the electron acceptor is chlorine, bromine and/or iodine which are liberated by heating an organic compound which is present in the composition and which forms Cl, Br and/or I.

18. Shaped articles, coatings and composite materials made of a composition according to claim 14.

19. A compound of the formula II or IIa

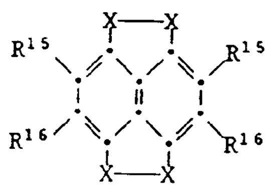 (II) 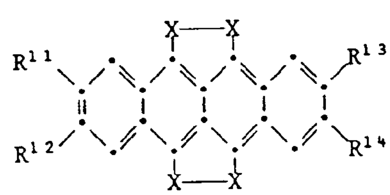 (IIa)

in which $R^{15}$ and $R^{16}$ are each phenylthio, 4-methylphenylthio, 4-methoxyphenylthio or 4-pyridylthio or in which $R^{15}$ and $R^{16}$ together are

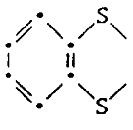

or

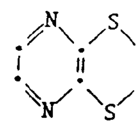

$R^{11}$ is $-CH_3$ and $R^{12}$, $R^{13}$ and $R^{14}$ are H, $R^{11}$ and $R^{12}$ are Cl or $CH_3$ and $R^{13}$ and $R^{14}$ are H, or $R^{11}$, $R^{12}$, $R^{13}$ and $R^{14}$ are $CH_3$ or F, and X is S, Se or Te.

**Claims for the following Contracting States: ES, GR**

1. A process for the preparation of a composition containing
a) a linear, branched or structurally crosslinked polymer and
b) at least one charge-transfer complex (CT complex) which is composed of at least one compound of the formula I or Ia and an electron acceptor

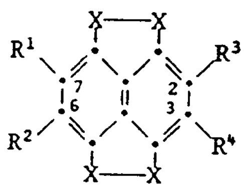

(I)

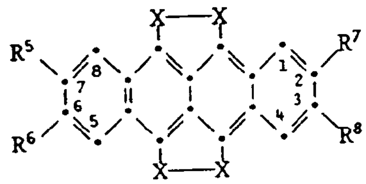

(Ia)

in which X is S, Se or Te, $R^1$, $R^2$, $R^3$ and $R^4$ independently of one another are a hydrogen atom or Cl or $R^1$ and $R^2$ and also $R^3$ and $R^4$ together are each

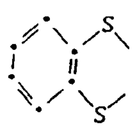

or

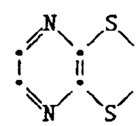

or $R^1$, $R^2$, $R^3$ and $R^4$ are each phenylthio, 4-methylphenylthio, 4-methoxyphenylthio or 4-pyridylthio, $R^5$, $R^6$, $R^7$ and $R^8$ independently of one another are H or F, $R^5$ is $CH_3$ and $R^6$, $R^7$ and $R^8$ are H, or $R^5$, $R^6$, $R^7$ and $R^8$ are $CH_3$, $R^5$ and $R^6$ are $CH_3$ or Cl, and $R^7$ and $R^8$ are H, or $R^5$ and $R^6$ are H, $R^7$ is $-COR^9$ and $R^8$ is H or $-COR^9$, or $R^5$ and $R^6$ are H and $R^7$ and $R^8$ together are -CO-O-CO or $-CO-NR^{10}-CO-$ in which $R^9$ is halide, -OH, $-NH_2$, $C_1$-$C_6$alkoxy, $C_2$-$C_6$hydroxyalkoxy, benzyloxy, phenoxy, cyclopentyloxy, cyclohexyloxy, $C_1$-$C_6$alkylamino or di-($C_1$-$C_6$alkyl)amino or is -OM in which M is a metal or ammonium cation, and $R^{10}$ is H, $C_1$-$C_{18}$alkyl, phenyl or benzyl, wherein $O_2$, a halogenating agent in the form of gas or vapour, an organic, halogen-containing compound which forms halogen when energy is supplied, or a salt of a cation having an oxidative action with non-nucleophilic anions is allowed to act as an electron acceptor on a composition containing
a) a linear, branched or structurally crosslinked polymer which is inert towards component b), and
b) a compound of the formula I or Ia or mixtures thereof.

2. A process according to claim 1, wherein the CT complex is present in an amount of 0.01 to 20% by weight, relative to the polymer.

3. A process according to claim 1, wherein the component b) is tetrathiotetracene, tetraselenotetracene, 2-fluorotetraselenotetracene or 2,3-difluorotetraselenotetracene.

4. A process according to claim 1, wherein the component b) is a mixture of compounds of the formulae I and Ia.

5. A process according to claim 1, wherein the composition contains, in addition, a solvent for a soluble and inert polymer and the component b).

6. A process according to claim 1, wherein the electron acceptor is a halogen-containing, organic compound which, when energy is supplied, splits off halogen.

7. A process according to claim 6, wherein the electron acceptor is chlorine, bromine and/or iodine which are liberated by heating an organic compound which is present in the composition and which forms Cl, Br and/or I.

8. A process according to claim 1, wherein the ratio of the electron acceptor to the component b) is 10:1

to 1:5.

9. A process according to claim 7, wherein the halogen-containing compound is a halogenated, saturated or unsaturated, aliphatic, cycloaliphatic, aliphatic-heterocyclic, aromatic or heteroaromatic, organic compound.

10. A process according to claim 6, wherein the halogen-containing, organic compound is tetrabromomethane, bromoform, trichlorobromomethane, hexachloropropene, hexachlorocyclopropane, hexachlorocyclopentadiene, hexachloroethane, N-chlorosuccinimide, octachloropropane, n-octachlorobutane, n-decachlorobutane, tetrabromoethane, hexabromoethane, tetrabromo-o-benzoquinone, N-bromosuccinimide, 2,4,4,6-tetrabromo-2,5-cyclohexadienone, hexabromobenzene, chloranil, hexachloroacetone, 1,4, 5,6,7,7-hexachloro-5-norbornene-2,3-dicarboxylic acid, 1,2,5,6,9,10-hexabromocyclododecane, tetrachloroethylene, perchlorocyclopentadiene, perchlorobutadiene, dichloroacetaldehyde diethylacetal, 1,4-dichloro-2-butene, 1,3-dichloro-2-butene, 3,4-dichloro-1-butene, tetrachlorocylcopropene, 1,3-dichloroacetone, 2,3, 5,6-hexachloro-p-xylene, 1,4-bis-(trichloromethyl)-benzene, 1,3-dibromopropane, 1,6-dibromohexane, ethyl 3-chloropropionate, 3-chlorotoluene, methyl 2-chloropropionate, 2-chloroacrylonitrile, ethyl trichloroacetate, 1,2,3-trichloropropane, 1,1,2-trichloroethane, butyl chloroformate, trichloroethylene, 2,3-dichloromaleic anhydride, 1,12-dibromododecane, α,α'-dibromo-p-xylene, α,α'-dichloro-o-xylene, phenacyl chloride or bromide, 1,10-dibromodecane, α,α'-dichloro-p-xylene, α,α'-dibromo-m-xylene, iodoacetonitrile, 2,3-dichloro-5,6-dicyanobenzoquinone, methyl 2,3-dichloropropionate, 1-bromo-2-chloroethane, 1-bromo-2-chloropropane, 2-bromoethyl chloroformate, ethyl iodoacetate, N-chlorosuccinimide, N-bromosuccinimide, N-iodosuccinimide, N-chlorophthalimide, N-bromophthalimide or N-iodophthalimide or mixtures thereof.

11. A process according to claim 6, wherein the halogen-containing, organic compound is liquid and is at the same time a solvent for the inert polymer and the component b).

12. A process according to claim 1, wherein the polymer is a thermosetting plastic, a thermoplastic or an elastomer.

## Revendications

### Revendications pour les Etats contractants suivants: AT, BE, CH, DE, FR, GB, IT, LI, NL, SE

1. Composition qui comprend :

a) un polymère linéaire, ramifié ou réticulé, inerte à l'égard du composant b) ci-dessous, avec

b) un composé de formule I ou Ia ou un mélange de ces composés

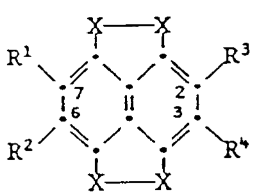 (I), 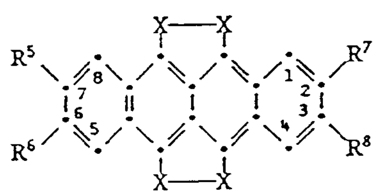 (Ia),

formules dans lesquelles X désigne S, Se ou Te, $R^1$, $R^2$, $R^3$ et $R^4$ désignent chacun, indépendamment les uns des autres, un atome d'hydrogène ou de chlore ou bien $R^1$ et $R^2$ et $R^3$ et $R^4$ forment ensemble, respectivement, un groupe

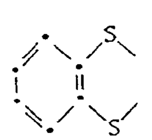 ou 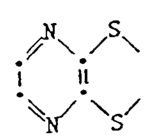 , ou encore

$R^1$, $R^2$, $R^3$ et $R^4$ sont chacun le groupe phénylthio, 4-méthyl- ou 4-méthoxy-phénylthio ou 4-pyridylthio, et $R^5$, $R^6$, $R^7$ et $R^8$ désignent chacun, indépendamment les uns des autres, l'hydrogène ou le fluor ou bien $R^5$ le groupe $CH_3$ et $R^6$, $R^7$ et $R^8$ chacun l'hydrogène, ou $R^5$, $R^6$, $R^7$ et $R^8$ sont le groupe $CH_3$, ou $R^5$ et $R^6$ le groupe $CH_3$ ou Cl et $R^7$ et $R^8$ l'hydrogène, ou encore $R^5$ et $R^6$ l'hydrogène, $R^7$ un groupe $-COR^9$ et $R^8$ l'hydrogène ou un groupe $-COR^9$, ou bien $R^5$ et $R^6$ l'hydrogène et $R^7$ et $R^8$ forment ensemble un groupe -CO-O-CO ou $-CO-NR^{10}-CO-$, $R^9$ désignant un halogène ou un groupe -OH, $-NH_2$, $C_1$-$C_6$-alcoxy, $C_2$-$C_6$-hydroalcoxy, benzyloxy, phénoxy, cyclopentyloxy, cyclohexyloxy, $C_1$-$C_6$-alkylamino ou di($C_1$-$C_6$-alkyl)ami-

no ou encore un groupe -OM, M étant un cation de métal ou un cation ammonium, et $R^{10}$ désignant l'hydrogène, un alkyle en $C_1$-$C_{18}$ ou le groupe phényle ou benzyle.

2. Composition selon la revendication 1, caractérisée en ce que la proportion du composant b) est de 0,01 à 20 % du poids du polymère.

3. Composition selon la revendication 1, caractérisée en ce que le composant b) est le tétrathiotétracène, le tétrasélénotétracène ou le 2-fluoro- ou 2,3-difluorotétrasélénotétracène.

4. Composition selon la revendication 1, caractérisée en ce qu'elle comprend un mélange des composés de formules I et Ia.

5. Composition selon la revendication 1, caractérisée en ce qu'elle contient également un solvant pour un polymère soluble et le composant b).

6. Composition selon la revendication 1, caractérisée en ce qu'elle contient également un accepteur d'électrons.

7. Composition selon la revendication 6, caractérisée en ce que l'accepteur d'électrons est un composé organique halogéné qui libère son halogène par un apport d'énergie.

8. Composition selon la revendication 6, caractérisée en ce que le rapport de l'accepteur d'électrons au composant b) est compris entre 10:1 et 1:5.

9. Composition selon la revendication 7, caractérisée en ce que le composé halogéné est un composé aliphatique saturé ou insaturé, cycloaliphatique, aliphatique-hétérocyclique, aromatique ou hétéroaromatique.

10. Composition selon la revendication 9, caractérisée en ce que le composé organique est chloré, bromé et/ou iodé.

11. Composition selon la revendication 7, caractérisée en ce que le composé organique halogéné est pris parmi les suivants : tétrabromométhane, bromoforme, trichlorobromo-méthane, hexachloropropène, hexachlorocyclopropane, hexachlorocyclopentadiène, hexachloréthane, N-chloro-succinimide, octachloropropane, n-octachlorobutane, n-décachlorobutane, tétrabromométhane, hexabromométhane, tétrabromo-o-benzoquinone, 2,4,4,6-tétrabromo-2,5-cyclohexa-diénone, hexabromobenzène, chloranile, hexachloracétone, acide 1,4,4,6,7,7-hexachloro-5-norbornène-2,3-dicarboxylique, 1,2,5,6,9,10-hexabromocyclododécane, tétrachloréthylène, perchlorocyclopentadiène, perchlorobutadiène, diéthylacétal du dichloracétaldéhyde, 1,4-dichloro-2-butène, 13-dichloro-2-butène, 3,4-dichloro-1-butène, tétrachlorocyclopropène, 1,3-dichloracétone, 2,3,5,6-hexachloro-p-xylène, 1,4-bis(trichlorométhyl)-benzène, 1,3-dibromopropane, 1,3-dibromopropane, 1,6-dibromohexane, 3-chloropropionate d'éthyle, 3-chlorotoluène, 2-chloropropionate de méthyle, 2-chloroacrylonitrile, trichloracétate d'éthyle, 1,2,3-trichloropropane, 1,1,2-trichloréthane, chloroformiate de butyle, trichloréthylène, anhydride de l'acide 2,3-dichloromaléique, 1,12-dibromododécane, $\alpha,\alpha'$-dibromo-p-xylène, $\alpha,\alpha'$-dichloro-o-xylène, chlorure et bromure de phénacyle, 1,10-dibromodécane, $\alpha,\alpha'$-dichloro-p-xylène, $\alpha,\alpha'$-dibromo-m-xylène, iodoacétonitrile, 2,3-dichloro-5,6-dicyanobenzoquinone, 2,3-dichloropropionate de méthyle, 1-bromo-2-chloréthane, 1-bromo-2-chloropropane, chloroformiate de 2-bromoéthyle, iodoacétate d'éthyle, succinimide et phtalimide chlorés, bromés ou iodés sur l'azote, ou leurs mélanges.

12. Composition selon la revendication 7, caractérisée en ce que le composé organique halogéné est liquide et constitue en même temps un solvant du polymère et du composant b).

13. Composition selon la revendication 1, caractérisée en ce que le polymère est un duroplaste, un thermoplaste ou un élastomère.

14. Composition qui comprend :

a) un polymère linéaire, ramifié ou réticulé, et

b) un réseau d'aiguilles cristallines conductrices de l'électricité dans la matrice du polymère, d'au moins un complexe de transfert de charges (complexe CT) d'un ou de plusieurs composés de formule I ou Ia selon la revendication 1 à l'état de chlorures, bromures ou iodures, ou avec comme accepteur d'électrons de l'oxygène ou un sel d'un cation oxydant à anions non-nucléophiles.

15. Composition selon la revendication 14, caractérisée en ce que le complexe CT est dans une proportion de 0,01 à 20 % du poids du polymère.

16. Procédé de préparation d'une composition selon la revendication 14 dans lequel on fait agir comme accepteur d'électrons, sur une composition conforme à la revendication 1, de l'oxygène, un agent d'halogénation gazeux ou vaporisé, un composé organique halogéné libérant son halogène par un apport d'énergie ou encore un sel d'un cation oxydant à anions non-nucléophiles.

17. Procédé selon la revendication 16, caractérisé en ce que l'accepteur d'électrons est du chlore, du brome et/ou de l'iode, qui sont libérés par un chauffage d'un composé organique contenu dans la composition, composé qui forme du chlore, du brome et/ou de l'iode par ce chauffage.

18. Objets moulés, revêtements et matériaux composites formés d'une composition selon la revendication 14.

19. Les composés de formules II et IIa ci-dessous :

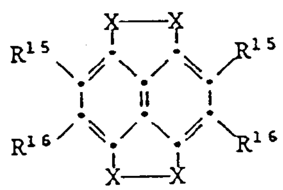 (II), 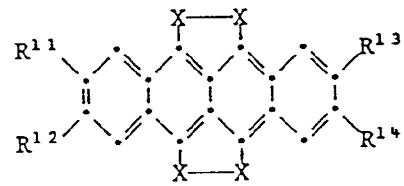 (IIa),

formules dans lesquelles $R^{15}$ et $R^{16}$ sont chacun un groupe phénylthio, 4-méthyl- ou 4-méthoxy-phénylthio ou 4-pyridylthio ou bien forment ensemble un groupe

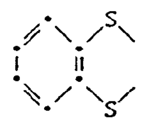 ou 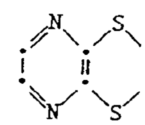

$R^{11}$ est le groupe-$CH_3$ et $R^{12}$, $R^{13}$ et $R^{14}$ désignent l'hydrogène, ou bien $R^{11}$ et $R^{12}$ sont le chlore ou le groupe-$CH_3$ et $R^{13}$ et $R^{14}$ l'hydrogène, ou encore $R^{11}$, $R^{12}$, $R^{13}$ et $R^{14}$ sont chacun le groupe -$CH_3$ ou le fluor, et X désigne S, Se ou Te.

**Revendications pour les Etats contractants suivants: ES, GR**

1. Procédé de préparation d'une composition qui comprend :
a) un polymère linéaire, ramifié ou réticulé avec
b) au moins un complexe de transfert de charges (complexe CT), formé d'un ou de plusieurs composés de formule I ou Ia ci-dessous et d'un accepteur d'électrons,

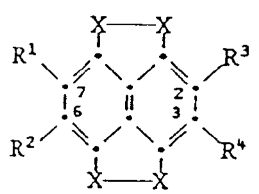 (I), 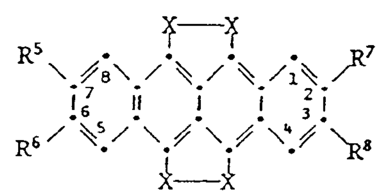 (Ia),

formules dans lesquelles X désigne S, Se ou Te, $R^1$, $R^2$, $R^3$ et $R^4$ désignent chacun, indépendamment les uns des autres, un atome d'hydrogène ou de chlore ou bien $R^1$ et $R^2$ et $R^3$ et $R^4$ forment ensemble, respectivement, un groupe

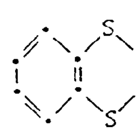 ou 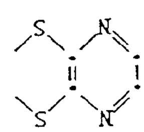 , ou encore

$R^1$, $R^2$, $R^3$ et $R^4$ sont chacun le groupe phénylthio, 4-méthyl- ou 4-méthoxy-phénylthio ou 4-pyridylthio, et $R^5$, $R^6$, $R^7$ et $R^8$ désignent chacun, indépendamment les uns des autres, l'hydrogène ou le fluor ou bien $R^5$ le groupe $CH_3$ et $R^6$, $R^7$ et $R^8$ chacun l'hydrogène, ou $R^5$, $R^6$, $R^7$ et $R^8$ sont le groupe $CH_3$, ou $R^5$ et $R^6$ le groupe $CH_3$ ou Cl et $R^7$ et $R^8$ l'hydrogène, ou encore $R^5$ et $R^6$ l'hydrogène, $R^7$ un groupe -$COR^9$ et $R^8$ l'hydrogène ou un groupe -$COR^9$, ou bien $R^5$ et $R^6$ l'hydrogène et $R^7$ et $R^8$ forment ensemble un groupe -CO-O-CO ou -CO-$NR^{10}$-CO-, $R^9$ désignant un halogène ou un groupe -OH, -$NH_2$, $C_1$-$C_6$-alcoxy, $C_2$-$C_6$-hydroalcoxy, benzyloxy, phénoxy, cyclopentyloxy, cyclohexyloxy, $C_1$-$C_6$-alkylamino ou di($C_1$-$C_6$-alkyl)amino ou encore un groupe -OM, M étant un cation de métal ou un cation ammonium, et $R^{10}$ désignant l'hydrogène, un alkyle en $C_1$-$C_{18}$ ou le groupe phényle ou benzyle, procédé caractérisé en ce que sur une composition qui comprend :
a) un polymère linéaire, ramifié ou réticulé, inerte à l'égard du composant b) ci-dessous, et

b) un composé de formule I ou Ia ou un mélange de ces composés, on fait agir comme accepteur d'électrons de l'oxygène, un agent d'halogénation gazeux ou vaporisé, un composé organique halogéné libérant son halogène par un apport d'énergie ou encore un sel d'un cation oxydant à anions non-nucléophiles.

2. Procédé selon la revendication 1, caractérisé en ce que la proportion du complexe CT est de 0,01 à 20 % du poids du polymère.

3. Procédé selon la revendication 1, caractérisé en ce que le composant b) est le tétrathiotétracène, le tétrasélénotétracène ou le 2-fluoro- ou 2,3-difluorotétrasélénotétracène.

4. Procédé selon la revendication 1, caractérisé en ce que le composant b) est constitué d'un mélange de composés de formules I et Ia.

5. Procédé selon la revendication 1, caractérisé en ce que la composition contient également un solvant pour un polymère soluble et inerte et pour le composant b).

6. Procédé selon la revendication 1, caractérisé en ce que l'accepteur d'électrons est un composé organique halogéné qui libère son halogène par un apport d'énergie.

7. Procédé selon la revendication 6, caractérisé en ce que l'accepteur d'électrons est du chlore, du brome ou de l'iode, qui sont libérés par chauffage d'un composé organique contenu dans la composition, composé formant du chlore, du brome et/ou de l'iode par chauffage.

8. Procédé selon la revendication 1, caractérisé en ce que le rapport de l'accepteur d'électrons au composant b) est compris entre 10:1 et 1:5.

9. Procédé selon la revendication 7, caractérisé en ce que le composé halogéné est un composé aliphatique saturé ou insaturé, cycloaliphatique, aliphatique-hétérocyclique, aromatique ou hétéroaromatique.

10. Procédé selon la revendication 6, caractérisé en ce que le composé organique halogéné est pris parmi les suivants : tétrabromométhane, bromoforme, trichlorobromo-méthane, hexachloropropène, hexachlorocyclopropane, hexachlorocyclopentadiène, hexachloréthane, N-chloro-succinimide, octachloropropane, n-octachlorobutane, n-décachlorobutane, tétrabromométhane, hexabromométhane, tétrabromo-o-benzoquinone, 2,4,4,6-tétrabromo-2,5-cyclohexa-diénone, hexabromobenzène, chloranile, hexachloracétone, acide 1,4,4,6,7,7-hexachloro-5-norbornène-2,3-dicarboxylique, 1,2,5,6,9,10-hexabromocyclododécane, tétrachloréthylène, perchlorocyclopentadiène, perchlorobutadiène, diéthylacétal du dichloracétaldéhyde, 1,4-dichloro-2-butène, 13-dichloro-2-butène, 3,4-dichloro-1-butène, tétrachlorocyclopropène, 1,3-dichloracétone, 2,3,5,6-hexachloro-p-xylène, 1,4-bis(trichlorométhyl)-benzène, 1,3-dibromopropane, 1,3-dibromopropane, 1,6-dibromohexane, 3-chloropropionate d'éthyle, 3-chlorotoluène, 2-chloropropionate de méthyle, 2-chloroacrylonitrile, trichloracétate d'éthyle, 1,2,3-trichloropropane, 1,1,2-trichloréthane, chloroformiate de butyle, trichloréthylène, anhydride de l'acide 2,3-dichloromaléique, 1,12-dibromododécane, $\alpha,\alpha'$-dibromo-p-xylène, $\alpha,\alpha'$-dichloro-o-xylène, chlorure et bromure de phénacyle, 1,10-dibromodécane, $\alpha,\alpha'$-dichloro-p-xylène, $\alpha,\alpha'$-dibromo-m-xylène, iodoacétonitrile, 2,3-dichloro-5,6-dicyanobenzoquinone, 2,3-dichloropropionate de méthyle, 1-bromo-2-chloréthane, 1-bromo-2-chloropropane, chloroformiate de 2-bromoéthyle, iodoacétate d'éthyle, succinimide et phtalimides chlorés, bromés ou iodés sur l'azote, ou leurs mélanges.

11. Procédé selon la revendication 6, caractérisé en ce que le composé organique halogéné est liquide et constitue en même temps un solvant du polymère inerte et du composant b).

12. Procédé selon la revendication 1, caractérisé en ce que le polymère est un duroplaste, un thermoplaste ou un élastomère.